# EUROPÄISCHE PATENTSCHRIFT

(11) **EP 3 727 532 B1**
(45) Veröffentlichungstag und Bekanntmachung des Hinweises auf die Patenterteilung: **12.10.2022**
(21) Anmeldenummer: 18815231.8
(22) Anmeldetag: 14.12.2018
(51) Int. Cl.: A61M 15/00, B05B 11/00

(54) **VORRICHTUNG ZUM VERSETZEN EINES INHALATORS IN EINEN AUSLÖSEBEREITEN ZUSTAND**
DEVICE FOR CHANGING AN INHALER TO A STATE READY FOR TRIGGERING
DISPOSITIF DE MISE EN PLACE D'UN INHALATEUR DANS UN ÉTAT PRÊT AU DÉCLENCHEMENT

(30) Priorität: 18.12.2017 EP 17208064
(43) Veröffentlichungstag der Anmeldung: 28.10.2020
(73) Patentinhaber: Boehringer Ingelheim International GmbH, 55216 Ingelheim am Rhein (DE)
(72) Erfinder: EICHER, Joachim Carl Herbert, 55216 Ingelheim am Rhein (DE); JUNG, Andree, 55216 Ingelheim am Rhein (DE)
(74) Vertreter: Simon, Elke Anna Maria
(86) Internationale Anmeldenummer: PCT/EP2018/084930
(87) Internationale Veröffentlichungsnummer: WO 2019/121386

(56) Entgegenhaltungen:
- WO-A1-92/00770
- WO-A1-2017/060328

## Beschreibung

Die vorliegende Erfindung betrifft eine Vorrichtung zum Versetzen eines Inhalators in einen auslösebereiten Zustand durch Hinzufügen von Energie zu einem Energiespeicher des Inhalators sowie ein System mit einer Vorrichtung und einem Inhalator.

Eine erfindungsgemäße Vorrichtung ist dazu ausgebildet, einem mechanischen Energiespeicher eines Inhalators Energie hinzuzufügen, insbesondere eine Feder bzw. Antriebsfeder zu spannen. Erfindungsgemäß wird der Inhalator durch das Hinzufügen von Energie zu dem Energiespeicher in einen auslösebereiten Zustand versetzt.

Ein "Inhalator" im Sinne der folgenden Erfindung ist vorzugsweise dazu ausgebildet, eine Substanz, beispielsweise ein Pulver oder ein Fluid, insbesondere eine Arzneimittelformulierung, zu zerstäuben bzw. in ein Aerosol zu überführen. Das erzeugte Aerosol kann dann von einem Benutzer bzw. Patienten eingeatmet bzw. inhaliert werden, insbesondere zur Behandlung einer Krankheit. Der Inhalator ist vorzugsweise ein portables bzw. mobiles Gerät.

Vorzugsweise weist ein Inhalator im Sinne der vorliegenden Erfindung einen Behälter als Reservoir für die zu zerstäubende Substanz auf, bevorzugt wobei der Behälter zur Abgabe der Substanz, insbesondere des Fluids, relativ zu einem Gehäuseteil - vorzugsweise axial - bewegbar ist. Besonders bevorzugt weist ein Inhalator einen insbesondere als Feder ausgebildeten Energiespeicher auf, wobei durch Freisetzung von Energie aus dem Energiespeicher bzw. durch Entspannung der Feder die zu zerstäubende Substanz unter Druck gesetzt und über eine Düse bzw. Austragsdüse zerstäubt bzw. als einatembares Aerosol ausgegeben wird. Weiter besonders bevorzugt ist durch eine relative Bewegung, insbesondere Verdrehung, zweier Gehäuseteile zueinander dem Energiespeicher Energie hinzufügbar bzw. die Feder spannbar. Ein solcher Inhalator ist beispielsweise aus der WO 2009/047173 A2 bekannt.

Ein "auslösebereiter Zustand" eines Inhalators ist ein Zustand, in dem eine Auslösung bzw. Zerstäubung des Inhalators unmittelbar erfolgen kann, beispielsweise durch Betätigung eines Auslöseelements wie einem Auslösemechanismus zur Auslösung einer mechanischen Druckerzeugung. Insbesondere ist ein auslösebereiter Zustand ein Zustand, in dem für die Auslösung bzw. Zerstäubung keine weiteren vorbereitenden Schritte oder Maßnahmen nötig sind. Besonders bevorzugt ist ein auslösebereiter Zustand ein Zustand, in dem durch Hinzufügen von Energie zu einem Energiespeicher bzw. durch Spannen einer Feder Energie für einen Druckerzeuger des Inhalators bereitgestellt ist, um die zu zerstäubende Substanz, insbesondere ein Fluid, zu zerstäuben.

Bei der Zerstäubung einer Substanz bzw. eines Fluids, insbesondere einer flüssigen Arzneimittelformulierung, mittels eines Inhalators der eingangs erwähnten Art soll eine möglichst genau definierte Menge an Wirkstoff in ein Aerosol zur Inhalation überführt werden. Das auf diese Weise erzeugte Aerosol soll sich durch einen kleinen Mittelwert der Tropfengröße bei einer schmalen Tropfengrößenverteilung und einem niedrigen Impuls bzw. eine niedrige Ausbreitungsgeschwindigkeit auszeichnen. Vorzugsweise weisen die so erzeugten Tropfen einen Tropfendurchmesser von weniger als 5 Mikrometer, insbesondere zwischen 2 Mikrometer und 5 Mikrometer, auf, da Tropfen dieser Größe bei der Inhalation in geeigneter Weise in Lungensystemen abgeschieden werden.

Der Begriff "Fluid" ist im Sinne der vorliegenden Erfindung vorzugsweise breit zu verstehen und auszulegen. Vorzugsweise handelt es sich bei dem Fluid um eine Flüssigkeit. Insbesondere umfasst der Begriff "Fluid" auch Wirkstoff-Lösungen, Dispersionen, Suspensionen oder dergleichen.

Unter dem Begriff "Aerosol" ist bei der vorliegenden Erfindung vorzugsweise eine wolkenartige oder nebelartige Ansammlung einer Vielzahl von flüssigen oder festen Schwebeteilchen einer vorzugsweise mittels eines Inhalators zerstäubten Substanz zu verstehen, vorzugsweise wobei die Schwebeteilchen eine geringe Geschwindigkeit und/oder zumindest im Wesentlichen ungerichtete Bewegungsrichtungen aufweisen. Ein "Aerosol" kann beispielsweise eine kegelförmige Teilchenwolke aufweisen oder bilden, insbesondere wobei die Hauptausbreitungsrichtung der Teilchenwolke zumindest im Wesentlichen der Hauptaustrittsrichtung bzw. Austrittsimpulsrichtung entspricht.

Bei dem aus der WO 2009/047173 A2 bekannten Inhalator muss eine Auslösung bzw. eine Zerstäubung zunächst vorbereitet werden, indem eine Antriebsfeder des Inhalators gespannt wird. Die anschließende Betätigung eines Auslöseknopfes bewirkt dann eine Zerstäubung bzw. Erzeugung eines Aerosols.

Zum Hinzufügen von Energie zu dem Energiespeicher, hier also insbesondere zum Spannen der Antriebsfeder, sind eine gewisse Handhabungskraft und - insbesondere zum Drehen der Gehäuseteile gegeneinander um 180° in einer durchgängigen Bewegung - motorische Koordination sowie insbesondere die Verwendung beider Hände erforderlich, was insbesondere für ältere bzw. geriatrische, geschwächte und/oder kranke Benutzer des Inhalators bzw. Patienten anstrengend bzw. problematisch sein kann. Unter Umständen ist es möglich, dass diese Personen es nur unter großer Anstrengung schaffen, den Inhalator ohne Hilfsmittel bzw. manuell durch Hinzufügen von Energie zu dem Energiespeicher in einen auslösebereiten Zustand zu versetzen und/oder das händische Hinzufügen von Energie zu dem Energiespeicher als schwierig oder problematisch empfinden. Dies kann zur Folge haben, dass ein benötigtes Medikament, das mittels des Inhalators inhaliert werden muss, nicht ohne Hilfe durch Pflegekräfte eingenommen werden kann oder wird.

Aus der WO 2017/060328 A1 ist ein System zum automatisierten Prüfen der Funktionsfähigkeit eines Inhalators als Teil einer Produktionsstraße bekannt. Das System ist zum Einsatz in einem Serienproduktionsprozess vorgesehen bzw. in einen Fertigungsprozess des Inhalators integriert und weist optional eine Spanneinrichtung zum Spannen eines Inhalators auf. Das System und die Spanneinrichtung sind nicht portabel und insbesondere weder manuell noch ohne Hilfsmittel durch einen Benutzer bzw. Patienten transportierbar. Auch wenn ebenfalls ein Inhalator gespannt wird, ist die Isolation dieser Funktion aus dem Systemzusammenhang nicht ohne Weiteres möglich, sodass die WO 2017/060238 A1 als fernliegender Stand der Technik zu betrachten ist.

Aus der WO 92/00770 ist eine portable Vorrichtung zum Versetzen eines Inhalators mit Treibgaskartusche in einen auslösebereiten Zustand durch Hinzufügen von Energie zu einem mechanischen Energiespeicher des Inhalators bekannt.

Vor diesem Hintergrund liegt der vorliegenden Erfindung die Aufgabe zugrunde, eine portable Vorrichtung und ein System hiermit anzugeben, mit der ein Inhalator, insbesondere durch Hinzufügen von Energie zu einem Energiespeicher bzw. durch Spannen einer Feder, in kraftschonender, komfortabler und/oder zuverlässiger Weise in einen auslösebereiten Zustand versetzt werden kann.

Die obige Aufgabe wird durch eine Vorrichtung gemäß Anspruch 1 oder ein System gemäß Anspruch 15 gelöst. Vorteilhafte Weiterbildungen sind Gegenstand der Unteransprüche.

Eine Vorrichtung im Sinne der vorliegenden Erfindung dient vorzugsweise zum Versetzen eines Inhalators in einen auslösebereiten Zustand durch Hinzufügen von Energie zu einem Energiespeicher, insbesondere durch Spannen einer Feder, des Inhalators.

Eine erfindungsgemäße Vorrichtung ist insbesondere eine mobile, autarke, batterie- bzw. akkumulatorbetriebene und/oder portable Vorrichtung bzw. eine Vorrichtung, die nicht fest an einem Ort installiert ist.

Die Vorrichtung ist vorzugsweise derart ausgebildet, dass ein Inhalator zum Versetzen in den auslösebereiten Zustand in die Vorrichtung einsetzbar und/oder mit der Vorrichtung mechanisch koppelbar ist.

Die Vorrichtung weist vorzugsweise einen motorischen Antrieb zum Bewegen zweier Gehäuseteile eines in die Vorrichtung eingesetzten und/oder mit der Vorrichtung gekoppelten Inhalators relativ zueinander auf, wobei durch die Bewegung der Gehäuseteile zueinander dem Energiespeicher des Inhalators Energie hinzufügbar bzw. die Feder des Inhalators spannbar ist. Ein manuelles Spannen durch einen Benutzer kann also entfallen, wodurch eine erleichterte und sichere Verwendung des Inhalators ermöglicht ist. Zudem kann so ein vollständiges bzw. ausreichendes Spannen der Feder sichergestellt werden, insbesondere wobei manuelle Einflüsse beim Spannen vermieden werden.

Der motorische Antrieb ermöglicht darüber hinaus eine einhändige Bedienung der Vorrichtung und/oder des Inhalators. Insbesondere entfällt durch die Verwendung der Vorrichtung das zweihändige manuelle Spannen der Feder bzw. Hinzufügen von Energie zu dem Energiespeicher. Dies bietet weiter den Vorteil, dass eine eventuelle (manuelle) Fehlbedienung des Inhalators vermieden wird.

Gemäß einem weiteren, auch unabhängig realisierbaren Aspekt weist die Vorrichtung zwei Kopplungseinrichtungen auf, wobei die Kopplungseinrichtungen jeweils mit einem von zwei Gehäuseteilen des Inhalators vorzugsweise mechanisch koppelbar sind, sodass mit den Kopplungseinrichtungen die zwei Gehäuseteile relativ zueinander bewegbar sind, wobei durch die Bewegung der Gehäuseteile zueinander dem Energiespeicher Energie hinzufügbar bzw. die Feder spannbar ist. Das Hinzufügen von Energie bzw. das Spannen ist so erleichtert, insbesondere automatisierbar, erfolgt also durch die Vorrichtung automatisch.

Erfindungsgemäß sind die Vorrichtung und/oder der Antrieb dazu ausgebildet, die Gehäuseteile gegeneinander zu verdrehen. Besonders bevorzugt sind die Vorrichtung und/oder der Antrieb dazu ausgebildet, die Gehäuseteile um mehr als 90° und/oder höchstens 360°, insbesondere um 180°, gegeneinander zu verdrehen. So ist ein leichtes und zuverlässiges Hinzufügen von Energie zu dem Energiespeicher ermöglicht.

Die Vorrichtung und/oder der Antrieb sind vorzugsweise derart ausgebildet, dass die durch sie eingebrachte Kraft bzw. Energie begrenzt ist. So ist eine Vorrichtung zum relativen Verdrehen von Gehäuseteilen zueinander vorzugsweise dazu ausgebildet, eine Krafteinleitung beim Erreichen eines vorgegebenen Maximaldrehmoments zu stoppen, vorzugsweise wobei das Maximaldrehmoment 0,5 Nm, 0,6 Nm oder 0,8 Nm beträgt. Auf diese Weise können eine Beschädigung oder Zerstörung des Inhalators, der Vorrichtung und/oder des Antriebs vermieden werden.

Der Antrieb weist erfindungsgemäß einen elektrischen Motor und/oder ein Getriebe auf. So ist ein einfaches, sicheres und zuverlässiges Hinzufügen von Energie zu dem Energiespeicher ermöglicht und die Bedienung des Inhalators und/oder der Vorrichtung erleichtert.

In einer bevorzugten Ausführungsform weist die Vorrichtung einen Sensor auf, der dazu ausgebildet ist, das Einsetzen des Inhalators, das Koppeln des Inhalators und/oder dessen Zustand bzw. Spannzustand zu detektieren. So ist ein zuverlässiges Spannen des Inhalators möglich und/oder kann ein unnötiges Spannen des Inhalators vermieden werden.

Der Ausdruck "Spannen des Inhalators" wird als Kurzform für "Hinzufügen von Energie zu dem Energiespeicher des Inhalators" verwendet und ist insbesondere synonym dazu zu verstehen.

Die Vorrichtung weist vorzugsweise eine Steuerung auf, die dazu ausgebildet ist, mit dem genannten Sensor das Einsetzen und/oder Koppeln des Inhalators und dessen Zustand bzw. Spannzustand zu detektieren. Besonders bevorzugt ist die Steuerung weiter dazu ausgebildet, in Abhängigkeit von dieser Detektion den Antrieb zu steuern, vorzugsweise sodass der Inhalator automatisch durch Hinzufügen von Energie zu dem Energiespeicher in den auslösebereiten Zustand versetzt wird.

Der Inhalator kann also von der Vorrichtung automatisch, insbesondere bei Einsetzen und/oder auf Knopfdruck bzw. durch Betätigen eines entsprechenden Schalters, in den auslösebereiten Zustand versetzt werden und/oder in diesem gehalten werden. Dies ist einer erleichterten Bedienung zuträglich, da ein Patient bzw. Benutzer des Inhalators so nach einer Benutzung des Inhalators diesen insbesondere manuell in die Vorrichtung einsetzen kann, wobei die Vorrichtung den Inhalator automatisch bzw. selbsttätig wieder in den auslösebereiten Zustand versetzt bzw. in diesem Zustand hält. So muss der Patient bzw. Benutzer für die nächste Inhalation bzw. Abgabe oder Auslösung den Inhalator lediglich aus der Vorrichtung entnehmen und kann unmittelbar bzw. ohne weitere Vorbereitung den Inhalator auslösen, die in dem Behälter enthaltende Substanz zerstäuben und/oder das erzeugte Aerosol inhalieren. So ist eine einfache und sichere Bedienung realisiert.

Die Vorrichtung kann eine Kommunikationseinrichtung zur vorzugsweise drahtlosen Kommunikation mit dem Inhalator und/oder einem externen System aufweisen. Die Kommunikationseinrichtung kann bedarfsweise mehrteilig ausgebildet sein und ist vorzugsweise dazu ausgebildet, Informationen über den Zustand des Inhalators und/oder dessen Benutzung zu senden und/oder zu empfangen. Die Bedienung des Inhalators und/oder der Vorrichtung kann so erleichtert bzw. weniger aufwendig gestaltet werden.

Die Vorrichtung weist vorzugsweise eine bevorzugt elektronisch betriebene Ausgabeeinrichtung, insbesondere ein Display und/oder einen Lautsprecher, zur bevorzugt optischen und/oder akustischen Ausgabe von Informationen über den Zustand des Inhalators und/oder dessen Benutzung auf. Dies ermöglicht eine erleichterte und/oder sichere Bedienung des Inhalators und/oder der Vorrichtung.

Es ist bevorzugt, dass die Vorrichtung eine Energieversorgungseinrichtung zur Versorgung der Vorrichtung mit (elektrischer) Energie aufweist. Die Vorrichtung bzw. Energieversorgungseinrichtung ist vorzugsweise zum Anschluss an ein Stromnetz ausgebildet. Besonders bevorzugt weist die Vorrichtung bzw. Energieversorgungseinrichtung einen vorzugsweise aufladbaren (elektrischen) Energiespeicher, insbesondere einen Akkumulator, auf. Dies ist einer zuverlässigen und/oder zumindest temporär stromnetzunabhängigen Funktion der Vorrichtung zuträglich.

Alternativ oder zusätzlich weist die Vorrichtung vorzugsweise eine Ladeeinrichtung zum Laden eines Energiespeichers des Inhalators auf. Dies ist einer zuverlässigen und/oder stromnetzunabhängigen Funktion des Inhalators zuträglich.

Besonders bevorzugt weist die Vorrichtung eine Aufnahme zum Einsetzen und/oder Halten des Inhalators auf. So ist eine einfache Bedienung bzw. Verwendung der Vorrichtung ermöglicht und die Vorrichtung kann ferner zur Lagerung des Inhalators dienen.

Ganz besonders bevorzugt ist die Vorrichtung dazu ausgebildet, dass durch insbesondere manuelles Einsetzen des Inhalators in die Aufnahme, beispielsweise durch einen Patienten bzw. Benutzer, der Antrieb mit einem der (beiden) Gehäuseteile verbunden wird und das andere Gehäuseteil - vorzugsweise formschlüssig - mit der Aufnahme verbunden wird, sodass das mit dem Antrieb verbundene Gehäuseteil relativ zu dem anderen Gehäuseteil bewegbar, vorzugsweise drehbar, ist und/oder das andere Gehäuseteil - zumindest während des Hinzufügens von Energie bzw. Spannens - unbeweglich und/oder vorzugsweise drehfest in der Aufnahme gehalten ist. So ist ein zuverlässiges Hinzufügen von Energie zu dem Energiespeicher bzw. Spannen der Feder ermöglicht.

Erfindungsgemäß betrifft die vorliegende Erfindung ein System mit einer Vorrichtung und einem Inhalator, wobei durch die Vorrichtung einem Energiespeicher des Inhalators durch eine relative Bewegung, insbesondere Verdrehung, zweier Gehäuseteile des Inhalators zueinander Energie hinzufügbar bzw. eine Feder spannbar ist.

Vorzugsweise weist der Inhalator einen Zähler zum Zählen von Benutzungen des Inhalators auf. Dies ist einer sicheren Benutzung des Inhalators bzw. Systems zuträglich. Ein Benutzer bzw. Patient kann so auf einfache Weise überprüfen, ob er/sie den Inhalator bzw. das System wie gewünscht oder vorgeschrieben benutzt. Insbesondere kann er/sie so sicherstellen, dass bzw. überprüfen, ob eine vorgeschriebene Anzahl von Dosen eines Medikaments eingenommen wird bzw. wurde. Der Zähler kann elektronisch oder mechanisch ausgebildet sein.

Vorzugsweise weisen die Vorrichtung und der Inhalator jeweils eine Kommunikationseinrichtung zur bevorzugt drahtlosen Kommunikation auf, sodass eine Datenverbindung zwischen den Kommunikationseinrichtungen der Vorrichtung und des Inhalators herstellbar ist.

Vorzugsweise ist der Zähler über eine Datenverbindung mit der Vorrichtung verbindbar. Die Vorrichtung wiederum ist vorzugsweise dazu ausgebildet, den Zählerstand zu empfangen, auszugeben und/oder an ein von dem Inhalator unterschiedliches externes Gerät zu übermitteln. Die Vorrichtung dient hier insbesondere als Koppelglied oder "Gateway" zur Bereitstellung von Benutzungsinformationen des Inhalators. So ist eine erleichterte Bedienung und/oder zuverlässige Funktionsweise des Systems ermöglicht.

Der Inhalator weist vorzugsweise einen bevorzugt mechanischen Druckerzeuger zur Förderung und Zerstäubung eines Fluids auf, wobei das Fluid bei Hinzufügen von Energie zu dem Energiespeicher bzw. Spannen der Feder in eine Druckkammer des Druckerzeugers gefördert wird und/oder wobei durch eine Entspannung des Energiespeichers bzw. der Feder das Fluid unter Druck gesetzt und über eine Düse als einatembares Aerosol ausgegeben wird. So kann auf einfache und zuverlässige Weise ein Aerosol der eingangs beschriebenen Art erzeugt werden. Bei derartigen Inhalatoren ist es besonders vorteilhaft, die nötige Energie des Energiespeichers bzw. Spannung der Feder bequem mittels der Vorrichtung zu erzeugen. Die Vorrichtung kann jedoch auch mit anderen Inhalatoren kombiniert werden.

Weitere Aspekte, Merkmale, Eigenschaften und Vorteile der vorliegenden Erfindung ergeben sich aus den Ansprüchen und der folgenden Beschreibung bevorzugter Ausführungsformen anhand der Zeichnung. Es zeigt:
- Fig. 1: einen schematischen Schnitt eines Inhalators im ungespannten Zustand;
- Fig. 2: einen schematischen, um 90° gegenüber Fig. 1 gedrehten Schnitt des Inhalators im gespannten Zustand;
- Fig. 3: eine perspektivische Außenansicht eines Inhalators;
- Fig. 4: eine perspektivische Darstellung einer vorschlagsgemäßen Vorrichtung gemäß einer ersten Ausführungsform mit einem darin eingesetzten Inhalator gemäß einer der Fig. 1 bis 3;
- Fig. 5: eine schematische, teiltransparente perspektivische Darstellung der Vorrichtung gemäß Fig. 3, wobei das Innenleben der Vorrichtung erkennbar ist;
- Fig. 6: eine schematische, teiltransparente Vorderansicht der Vorrichtung gemäß Fig. 4 und 5 mit eingesetztem Inhalator;
- Fig. 7: eine schematische, teiltransparente perspektivische Darstellung der Vorrichtung gemäß einer weiteren Ausführungsform;
- Fig. 8: eine vereinfachte, blockschaltbildartige schematische Darstellung der Vorrichtung und des Inhalators gemäß Fig. 4 und 6;
- Fig. 9: eine Vorderansicht eines Inhalators gemäß einer weiteren Ausführungsform;
- Fig. 10: eine Vorderansicht einer vorschlagsgemäßen Vorrichtung gemäß einer weiteren Ausführungsform mit eingesetztem Inhalator gemäß der Ausführungsform aus Fig. 9; und
- Fig. 11: einen Schnitt durch die Vorrichtung mit eingesetztem Inhalator entlang der Linie XI-XI aus Fig. 10.

In den teilweise nicht maßstabsgerechten, nur schematischen Figuren werden für gleiche oder ähnliche Teile dieselben Bezugszeichen verwendet, wobei entsprechende oder vergleichbare Eigenschaften und Vorteile erreicht werden können, auch wenn von einer wiederholten Beschreibung der Übersichtlichkeit halber abgesehen wird.

Nachfolgend wird zunächst eine besonders bevorzugte Ausführungsform eines Inhalators 1 beschrieben, der mit einer vorschlagsgemäßen Vorrichtung 23 in einen auslösebereiten Zustand versetzbar ist. Im Anschluss daran wird auf die Vorrichtung 23 selbst eingegangen.

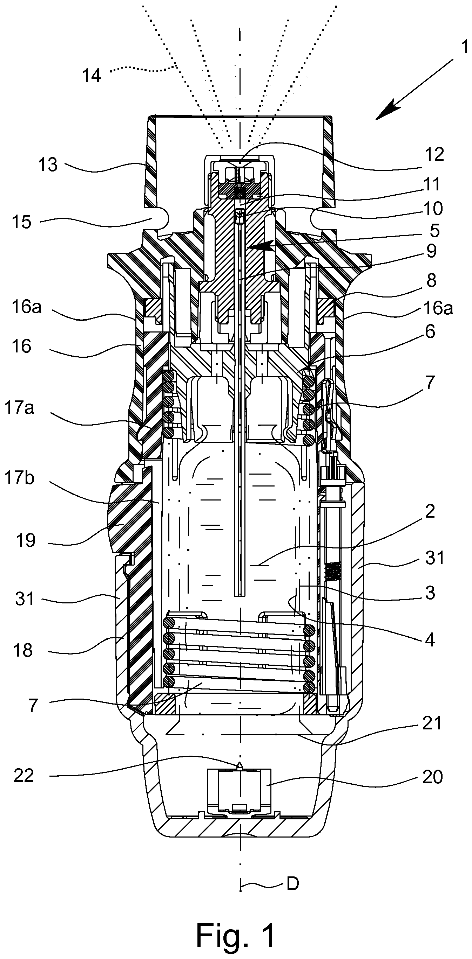

Die Fig. 1 bis 3 zeigen einen Inhalator 1 zur Zerstäubung eines Fluids 2, insbesondere eines hochwirksamen Arzneimittels oder dgl., wie er beispielsweise in die erfindungsgemäße Vorrichtung 23 eingesetzt und/oder mit dieser gekoppelt wird.

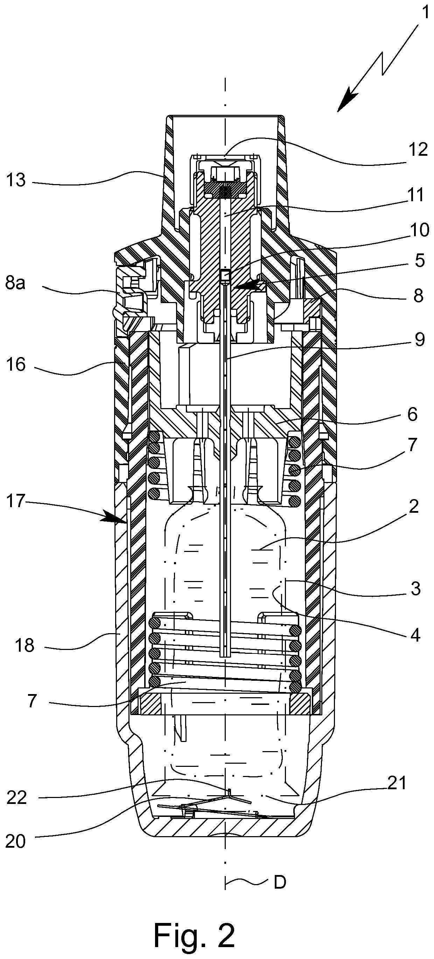

Fig. 1 und Fig. 2 zeigen dabei den Inhalator 1 in einer schematischen Darstellung im nicht auslösebereiten oder ungespannten Zustand (Fig. 1) und im auslösebereiten oder gespannten Zustand (Fig. 2). Die Bezeichnungen "ungespannter" und "gespannter Zustand" bezeichnen hierbei den Zustand des im Inhalator 1 enthaltenen, vorzugsweise durch eine Feder bzw. Antriebsfeder gebildeten, Energiespeichers 7. Im "ungespannten Zustand" (Fig. 1) ist der Inhalator 1 also nicht auslösebereit und im "gespannten Zustand" (Fig. 2) ist der Inhalator 1 auslösebereit. Der gespannte bzw. ungespannte Zustand bzw. Spannzustand des Inhalators 1 ist also insbesondere der (Spann-)Zustand des Energiespeichers 7 bzw. der Antriebsfeder des Inhalators 1.

Der Energiespeicher 7 bzw. die Antriebsfeder ist vorzugsweise derart in dem Inhalator 1 eingebaut, dass der Energiespeicher 7 bzw. die Antriebsfeder bereits vorgespannt bzw. teilweise komprimiert ist. Der ungespannte Zustand ist vorzugsweise ein Zustand, in dem der Inhalator 1 nicht auslösebereit ist, wobei aber der Energiespeicher 7 bzw. die Antriebsfeder bereits vorgespannt bzw. teilweise komprimiert ist.

Unter dem Ausdruck "Spannen" der Feder bzw. Antriebsfeder ist insbesondere eine Auslenkung der Feder bzw. eine Veränderung der Federlänge der Feder zu verstehen, bei der eine Federkraft bzw. Rückstellkraft erzeugt bzw. vergrößert wird. Mit anderen Worten wird durch das Spannen der Feder Energie, insbesondere Spannenergie, zu der Feder hinzugefügt und (insbesondere als potentielle Energie) in dieser gespeichert. Grundsätzlich umfasst der Begriff "Spannen" der Feder sowohl eine Verlängerung bzw. Streckung der Feder als auch eine Verkürzung bzw. Komprimierung der Feder. Bei dem bevorzugten Ausführungsbeispiel des Inhalators 1 wird die Feder bzw. der Energiespeicher 7 vorzugsweise durch Zusammendrücken bzw. Komprimieren gespannt.

Der Inhalator 1 ist erfindungsgemäß als tragbarer, portabler und/oder mobiler Inhalator 1 ausgebildet.

Bei Zerstäubung der Substanz bzw. des Fluids 2, vorzugsweise einer Flüssigkeit, insbesondere eines Arzneimittels, mittels des Inhalators 1 wird vorzugsweise ein Aerosol 14 gebildet, insbesondere wobei das Aerosol 14 von einem nicht dargestellten Benutzer eingeatmet bzw. inhaliert werden kann.

Üblicherweise erfolgt das Inhalieren wenigstens einmal täglich, insbesondere mehrmals täglich, vorzugsweise in vorbestimmten Zeitabständen, beispielsweise in Abhängigkeit von der Erkrankung des Patienten.

Bei dem Aerosol 14 handelt es sich vorzugsweise um ein Partikel-Luft-Gemisch mit festen und/oder flüssigen Partikeln, die besonders bevorzugt lungengängig sind, also insbesondere jedenfalls teilweise oder im Durchschnitt kleiner als 5 µm im Durchmesser sind.

Der Inhalator 1 ist vorzugsweise treibgasfrei, arbeitet also vorzugsweise ohne Treibgas. Stattdessen arbeitet der Inhalator 1 vorzugsweise mit einem (mechanischen) Mechanismus zur Erzeugung des Aerosols 14. Insbesondere handelt es sich hierbei um eine mechanisch betriebene Pumpe, die das Fluid 2 unter Druck setzt, wodurch das Aerosol 14 erzeugt wird. Grundsätzlich kann der Inhalator 1 jedoch auch auf andere, bevorzugt mechanische, Weise angetrieben werden.

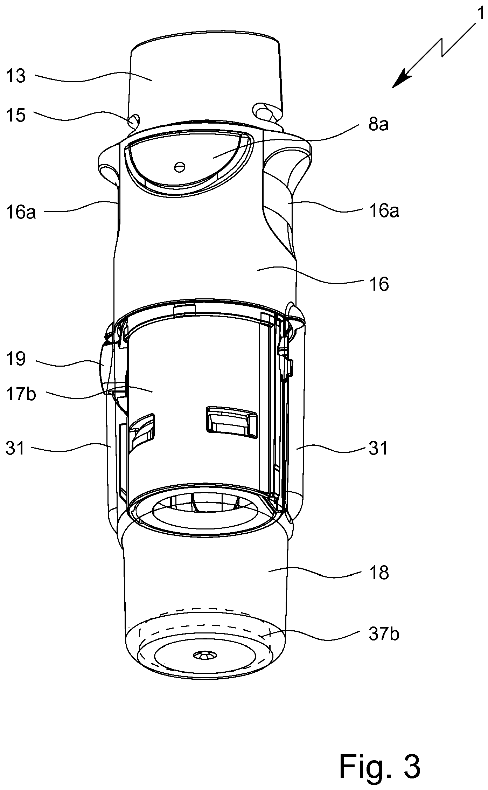

Der Inhalator 1 weist einen vorzugsweise einsetzbaren und vorzugsweise auswechselbaren Behälter 3 mit dem Fluid 2 auf. Der Behälter 3 bildet vorzugsweise ein Reservoir für das zu zerstäubende Fluid 2. In Fig. 3 ist der Inhalator 1 ohne den Behälter 3 dargestellt.

Vorzugsweise enthält der Behälter 3 eine ausreichende Menge an Fluid 2 bzw. Wirkstoff, um beispielsweise bis zu 200 Dosiereinheiten zur Verfügung stellen zu können, also beispielsweise bis zu 200 Zerstäubungen oder Anwendungen zu ermöglichen. Ein typischer Behälter 3, wie in der WO 96/06011 A2 offenbart, nimmt ein Volumen von ca. 2 ml bis 10 ml auf.

Der Behälter 3 ist vorzugsweise zumindest im Wesentlichen zylindrisch bzw. kartuschenartig ausgebildet und von unten, nach Öffnen des Inhalators 1, in diesen einsetzbar und ggf. wechselbar.

Der Behälter 3 ist vorzugsweise zumindest im Wesentlichen starr ausgebildet. Besonders bevorzugt ist der Behälter 3 aus Kunststoff, insbesondere thermoplastischem Kunststoff, ganz besonders bevorzugt Polypropylen, hergestellt. Vorzugsweise weist der Behälter 3 an seinem Boden eine ebene Fläche auf oder hat der Behälter 3 einen ebenen Behälterboden 21.

Optional weist der Behälter 3 eine metallische und/oder reflektierende Außenhülle und/oder einen metallischen und/oder reflektierenden Behälterboden 21 oder eine metallische und/oder reflektierende (äußere) Beschichtung am Behälterboden 21 auf.

Vorzugsweise ist das Fluid 2 in einem von einem kollabierbaren Beutel gebildeten Fluidraum 4 im Behälter 3 aufgenommen.

Der Inhalator 1 weist vorzugsweise ferner einen vorzugsweise mechanischen Druckerzeuger 5 zur Förderung und Zerstäubung des Fluids 2, insbesondere jeweils in einer vorbestimmten, ggf. einstellbaren, Dosiermenge auf.

Der Druckerzeuger 5 weist vorzugsweise einen - im Darstellungsbeispiel nur teilweise dargestellten - Energiespeicher 7 zum Antrieb des Druckerzeugers 5 auf. Der Energiespeicher 7 ist hierbei vorzugsweise dazu ausgebildet, Energie zu speichern und an den Druckerzeuger 5 abzugeben, sodass der Druckerzeuger 5 das Fluid 2 unter Druck setzt, wodurch aus dem Fluid 2 das Aerosol gebildet wird. Der Druckerzeuger 5 wird also mit dem Energiespeicher 7 bzw. der durch den Energiespeicher 7 zur Verfügung gestellten Energie angetrieben.

Vorzugsweise ist der Energiespeicher 7 als Feder bzw. Schraubenfeder ausgebildet. Das Hinzufügen von Energie zu dem Energiespeicher 7 bzw. Spannen der Feder erfolgt insbesondere durch Zusammendrücken des Energiespeichers 7 bzw. der Feder.

Der Druckerzeuger 5 weist vorzugsweise eine Halterung 6 für den Behälter 3, den zugeordneten, nur teilweise dargestellten Energiespeicher 7 mit einem zur Entsperrung - direkt oder vorzugsweise über eine Auslösetaste 8a - manuell betätigbaren Sperrelement 8, ein Förderrohr 9 mit einem Rückschlagventil 10, eine Druckkammer 11 und eine Austragsdüse 12 im Bereich eines Mundstücks 13 auf. Der Behälter 3 wird vorzugsweise über die Halterung 6, insbesondere rastend, so in dem Inhalator 1 fixiert, dass das Förderrohr 9 in den Behälter 3 eintaucht. Die Halterung 6 kann dabei derart ausgebildet sein, dass der Behälter 3 gelöst und ausgetauscht werden kann.

Beim axialen Spannen des Energiespeichers 7 bzw. der Feder wird die Halterung 6 mit dem Behälter 3 und dem Förderrohr 9 bei den Darstellungen nach unten bewegt und das Fluid 2 aus dem Behälter 3 über das Rückschlagventil 10 in die Druckkammer 11 des Druckerzeugers 5 gesaugt.

Beim anschließenden Entspannen des Energiespeichers 7 bzw. der Feder nach Betätigung des Sperrelements 8 wird das Fluid 2 in der Druckkammer 11 unter Druck gesetzt, indem das Förderrohr 9 mit seinem nun geschlossenen Rückschlagventil 10 durch Entspannen des Energiespeichers 7 bzw. der Feder wieder nach oben bewegt wird und nun als Druckstempel dient. Dieser Druck treibt das

Fluid 2 durch die Austragsdüse 12 aus, wobei es in das Aerosol 14 zerstäubt wird, wie in Fig. 1 angedeutet.

Vorzugsweise ist das Förderrohr 9, insbesondere über die Halterung 6, in Gebrauchslage gegenüber dem Behälter 3 festgelegt. Es ist also insbesondere vorgesehen, dass eine (axiale) Bewegung des Förderrohrs 9 einer (axialen) Bewegung des Behälters 3 entspricht.

Wenn das Förderrohr 9 bzw. das Rückschlagventil 10 als Druckstempel fungiert, korrespondiert eine Bewegung des Förderrohrs 9 bzw. des Behälters 3 zu einem in der Druckkammer 11 verdrängten Volumen bzw. einer ausgetragenen oder austragbaren Fluidmenge.

Ein nicht dargestellter Benutzer bzw. Patient kann das zerstäubte Fluid 2 bzw. das Aerosol 14 inhalieren, vorzugsweise wobei Zuluft über mindestens eine Zuluftöffnung 15 in das Mundstück 13 saugbar ist.

Der Inhalator 1 weist erfindungsgemäß zueinander drehbare Gehäuseteile 16, 17, 18 auf. Ferner ist der Inhalator 1 erfindungsgemäß dazu ausgebildet, durch Verdrehen der Gehäuseteile 16, 17, 18 zueinander dem Energiespeicher 7 Energie hinzuzufügen bzw. die Feder zu spannen, insbesondere also die Feder bzw. Antriebsfeder zu komprimieren.

Der Inhalator 1 weist im Darstellungsbeispiel vorzugsweise ein Gehäuseteil 16 und ein demgegenüber drehbares Innenteil 17 bzw. inneres Gehäuseteil (Fig. 2) mit einem oberen Teil 17a und einem unteren Teil 17b (Fig. 1) auf. An dem Innenteil 17 ist vorzugsweise ein Gehäuseunterteil bzw. (unteres) Gehäuseteil 18 bzw. eine Kappe vorzugsweise mittels eines Halteelementes 19 lösbar befestigt, insbesondere aufgesteckt. Vorzugsweise ist das Innenteil 17 bzw. innere Gehäuseteil mittels des Gehäuseunterteils bzw. Gehäuseteils 18 gegenüber dem Gehäuseteil 16 manuell verdrehbar.

In Fig. 3 ist das Gehäuseteil 18 durchsichtig dargestellt. Vorzugsweise ist das Gehäuseteil 18 durchsichtig.

Zum Einsetzen und/oder Auswechseln des Behälters 3 ist das Gehäuseunterteil bzw. untere Gehäuseteil 18 vorzugsweise vom Inhalator 1 lösbar. Der Behälter 3 kann jedoch auch unauswechselbar bzw. gegen Entnahme gesichert sein

Das Gehäuseteil 18 kann gegen das Gehäuseteil 16 gedreht bzw. relativ zu dem Gehäuseteil 16 verdreht werden, vorzugsweise wobei es den in der Darstellung unteren Teil 17b des Innenteils 17 mitnimmt.

Insbesondere ist das Gehäuseteil 18 drehfest an dem Gehäuseteil bzw. Innenteil 17 angeordnet, vorzugsweise mittels einer formschlüssigen Verbindung.

Das Gehäuseteil bzw. Innenteil 17 kann also ebenfalls gegen das Gehäuseteil 16 gedreht bzw. relativ zu dem Gehäuseteil 16 verdreht werden.

Das Drehen der Gehäuseteile 16, 17, 18 gegeneinander bzw. das relative Verdrehen der Gehäuseteile 16, 17, 18 zueinander erfolgt um eine Rotationsachse oder Drehachse D des Inhalators 1. Die Drehachse D bildet vorzugsweise eine Längsachse, Mittelachse und/oder Symmetrieachse des Inhalators 1.

Nachfolgend wird unter einer "axialen" Bewegung bzw. Richtung eine Bewegung bzw. Richtung verstanden, die identisch mit der Drehachse D ist und/oder parallel zu oder entlang dieser verläuft. Auch der Begriff "radial" bezieht sich entsprechend jeweils auf die Drehachse D.

Durch das Drehen des Gehäuseteils 16 bzw. des Innenteils 17 wird der Energiespeicher 7 bzw. die Feder über ein nicht dargestelltes, auf die Halterung 6 wirkendes Getriebe in axialer Richtung gespannt, insbesondere zusammengedrückt. Mit dem Spannen wird der Behälter 3 vorzugsweise axial nach unten bewegt, bis der Behälter 3 eine in Fig. 2 angedeutete Endlage annimmt ("unten" bezieht sich hierbei auf die in den Figuren dargestellte Lage des Inhalators 1, "unten" am Inhalator 1 ist hierbei das mundstückferne Ende des Inhalators 1). In diesem Zustand ist der Energiespeicher 7 bzw. die Feder gespannt bzw. befindet sich der Inhalator 1 im gespannten Zustand. Während des Zerstäubungsvorgangs wird der Behälter 3 vorzugsweise von dem Energiespeicher 7 bzw. der Feder wieder (nach oben, d. h. hier in Richtung des Mundstücks 13) in seine Ausgangslage zurückbewegt.

Der Behälter 3 führt vorzugsweise eine axiale Bewegung oder Hubbewegung während dem Hinzufügen von Energie bzw. während des Spannvorgangs bzw. zur Fluidentnahme und/oder während der Zerstäubung bzw. Abgabe des Fluids 2 aus.

Insbesondere steht die axiale Position des Behälters 3 also in direktem Zusammenhang mit dem Zustand bzw. Spannzustand des Inhalators 1 bzw. Energiespeichers 7 bzw. ist die axiale Position des Behälters 3 mit dem Zustand bzw. Spannzustand korreliert. Im in Fig. 1 dargestellten ungespannten Zustand befindet sich der Behälter 3 vorzugsweise in der aus Fig. 1 ersichtlichen oberen axialen Position. Im gespannten Zustand befindet sich der Behälter 3 vorzugsweise in der aus Fig. 2 ersichtlichen unteren axialen Position.

Vorzugsweise kommt beim erstmaligen Spannen eine axial wirkende, im Gehäuseteil 18 angeordnete Feder 20 am Behälterboden 21 zur Anlage und sticht mit einem Anstechelement 22 den Behälter 3 bzw. eine bodenseitige Versiegelung bei der erstmaligen Anlage zur Belüftung an. Die Feder 20 mit dem Anstechelement 22 ist bei der Darstellung in Fig. 3 weggelassen.

Entlang der Drehachse D beträgt die Länge des Inhalator 1 vorzugsweise zwischen 6 cm und 13 cm, bevorzugt etwa 10 cm, insbesondere gemessen vom Boden des unteren Gehäuseteils 18 bis zum oberen Ende des Mundstücks 13. Die Länge des unteren Gehäuseteils 18 entlang der Drehachse D beträgt vorzugsweise zwischen 3 cm und 7 cm, bevorzugt etwa 5 cm.

Vorzugsweise weist der Inhalator 1 senkrecht zur Drehachse D zumindest im Wesentlichen einen rundlichen, insbesondere ovalen und/oder kreisförmigen, Querschnitt auf. Der Durchmesser des Inhalators 1 senkrecht zur Drehachse D beträgt vorzugsweise zwischen 2 cm und 5 cm, bevorzugt etwa 3 cm. Im Bereich der Abflachungen 16a und/oder der Kopplungsabschnitte 31 bzw. durch die Abflachungen 16a und/oder die Kopplungsabschnitte 31 kann der Querschnitt des oberen Gehäuseteils 16 und/oder des unteren Gehäuseteils 18 zumindest abschnittsweise von einer rundlichen Form bzw. Kreisform abweichen.

Der Inhalator 1 weist vorzugsweise eine in den Figuren nicht dargestellte Blockiervorrichtung auf, mit der die weitere Benutzung oder Betätigung des Inhalators 1 nach einer vorgegebenen Anzahl von Betätigungen verhindert wird. Vorzugsweise ist die Blockiervorrichtung wie in der WO 2004/024340 A1 beschrieben ausgebildet. Die Blockiervorrichtung weist vorzugsweise eine vorgespannte Blattfeder auf, die zwischen dem oberen Gehäuseteil 16 und dem inneren Gehäuseteil 17 angeordnet ist und nach einer vorbestimmten Anzahl von Betätigungen des Inhalators 1 eine weitere Betätigung, insbesondere ein Drehen der Gehäuseteile 16, 17, 18 gegeneinander, blockiert. Das Blockieren erfolgt insbesondere dadurch, dass die Blattfeder nach einer vorgegebenen Anzahl von Betätigungen durch einen Stößel axial verschoben wird, sodass sie in zwei dazu vorgesehene Aussparungen der Gehäuseteile 16 und 17 eingreift bzw. zwischen diesen Aussparungen angeordnet ist und somit ein weiteres bzw. erneutes Verdrehen der Gehäuseteile 16 und 17 zueinander blockiert.

Die Gehäuseteile 16, 17, 18 können bei aktivierter Blockiervorrichtung vorzugsweise nur mit großem Kraftaufwand gegeneinander gedreht werden, wobei jedoch der Inhalator 1 selbst zerstört und/oder unbrauchbar wird. Die bei aktivierter Blockiervorrichtung zum Verdrehen der Gehäuseteile 16, 17, 18 benötigte Energie oder Arbeit bzw. das dazu benötigte Drehmoment beträgt vorzugsweise etwa 3 Nm.

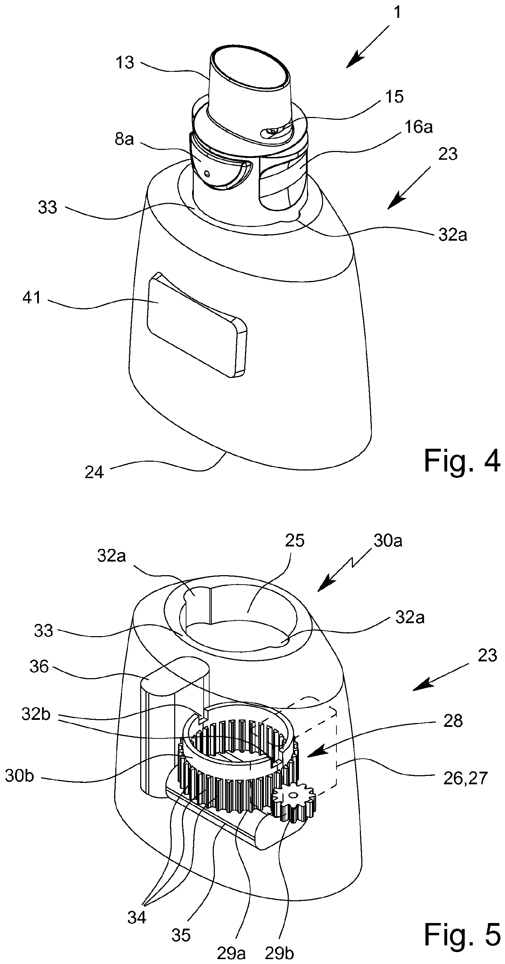

Die Fig. 4 bis 6 zeigen in verschiedenen Darstellungen eine vorschlagsgemäße Vorrichtung 23 zum Versetzen eines Inhalators in den auslösebereiten Zustand, insbesondere durch Hinzufügen von Energie zu einem Energiespeicher 7 bzw. durch Spannen einer Feder des Inhalators.

Es ist anzumerken, dass die nachfolgend beschriebene Vorrichtung 23 grundsätzlich unabhängig, d.h. als separate Einheit, von dem zuvor beschriebenen Inhalator 1 realisierbar ist. Insbesondere ist die Vorrichtung 23 nicht auf die Verwendung mit einem individuellen Inhalator 1 beschränkt, sondern kann mit baugleichen Geräten (z.B. nach Leerung oder Erreichen der Betriebsdauer des Inhalators 1) weiter verwendet werden. Vorzugsweise kann die Vorrichtung 23 dazu ausgebildet sein, im Zusammenhang mit anderen Inhalatoren, die nicht der gerade beschriebenen Ausführungsform entsprechen oder nicht exakt baugleich sind, betrieben zu werden, wobei insbesondere auch der Einsatz von Wechseleinsätzen möglich ist, sodass die Vorrichtung 23 mit unterschiedlichen Inhalatoren 1, die unterschiedlich ausgebildete Kopplungsabschnitte 31 aufweisen, formschlüssig koppelbar ist. Grundsätzlich sind einzelne Aspekte der nachfolgend beschriebenen Vorrichtung 23 unabhängig voneinander und/oder unabhängig von dem Inhalator 1 realisierbar.

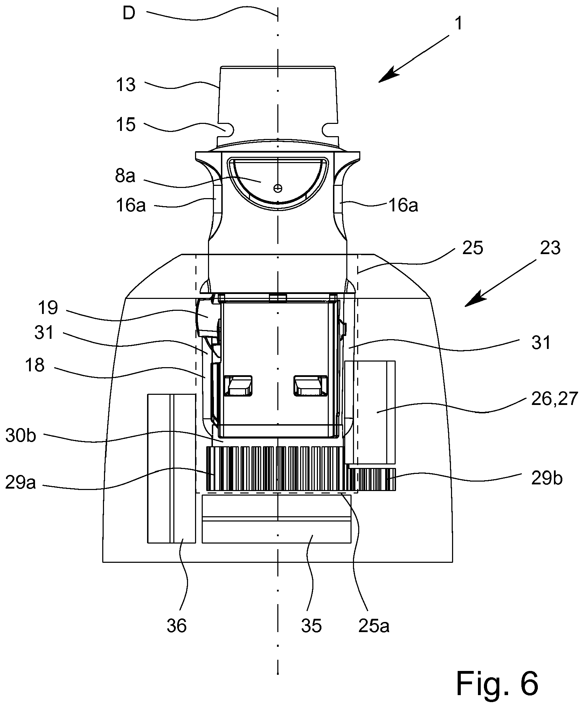

Bevorzugt ist die Vorrichtung 23 jedoch zum Spannen der Feder des zuvor beschriebenen Inhalators 1 ausgebildet. Der Inhalator 1 und die Vorrichtung 23 bilden vorzugsweise ein System, wie insbesondere in Fig. 6 gezeigt.

Vorzugsweise ist die Vorrichtung 23 derart ausgebildet, dass der Inhalator 1 zur Verwendung im Zusammenhang mit der Vorrichtung 23 nicht modifiziert werden muss und/oder dass der Inhalator 1 auch ohne die Vorrichtung 23 uneingeschränkt weiter benutzbar ist, insbesondere weiter manuell in den auslösebereiten Zustand versetzbar ist. Dies ist bei einem Ausfall oder einer Beschädigung der Vorrichtung 23 insbesondere der Bediensicherheit zuträglich.

Erfindungsgemäß ist die Vorrichtung 23 kompakt, portabel, tragbar und/oder mobil ausgebildet. Die Vorrichtung 23 ist also beispielsweise als Auftischgerät bzw. Tischgerät oder Handgerät ausgebildet. Insbesondere ist die Vorrichtung 23 als eine Art Dockingstation, Tischspannstation und/oder Ladestation für den Inhalator 1 ausgebildet. Die Vorrichtung 23 kann z. B. ähnlich einer Ladestation für ein Handy, ein Mobilteil eines Festnetztelefons oder eine elektrische Zahnbürste ausgebildet sein.

Erfindungsgemäß ist eine portable, tragbare und/oder mobile Vorrichtung 23 eine Vorrichtung 23, die zum persönlichen Gebrauch durch einen Patienten bzw. Nutzer des Inhalators 1 bestimmt ist und nicht industriell bzw. bei der Produktion und/oder Funktionsprüfung des Inhalators 1 eingesetzt wird. Die Vorrichtung 23 ist erfindungsgemäß manuell und ohne Hilfsmittel von einem Benutzer bzw. Patienten transportierbar.

Die Vorrichtung 23 ist vorzugsweise nicht fest an einem Ort installiert, befestigt oder verankert. Insbesondere ist vorgesehen, die Vorrichtung 23 für den Heimgebrauch und/oder nicht industriell einzusetzen.

Die Vorrichtung 23 ist hierbei vorzugsweise dazu ausgebildet, netzunabhängig betrieben zu werden oder betreibbar zu sein. Insbesondere weist die Vorrichtung hierzu einen Energiespeicher wie einen Akkumulator, eine Batterie oder dergleichen auf.

Die Vorrichtung 23 wiegt vorzugsweise weniger als 1 kg, insbesondere weniger als 0,5 kg. Die Vorrichtung 23 weist vorzugsweise eine maximale Erstreckung von weniger als 25 cm, insbesondere weniger als 20 cm, besonders bevorzugt weniger als 15 cm, und/oder ein Volumen von weniger als 2 dm³, insbesondere weniger als 1 dm³, auf.

Die Vorrichtung 23 ist insbesondere auf einem Tisch, Regal, Sideboard, Beistelltisch, Nachttisch und/oder in einem Schrank o. dgl. abstellbar oder lagerbar.

Es ist vorzugsweise vorgesehen, dass der Inhalator 1 dauerhaft bzw. über einen längeren Zeitraum in der Vorrichtung 23 gehalten, gelagert oder aufbewahrt wird und/oder nur zur Inhalation oder einer sonstigen Benutzung bzw. Betätigung des Inhalators 1 aus der Vorrichtung 23 entnommen oder von dieser getrennt wird.

Vorzugsweise ist die Vorrichtung 23 dazu ausgebildet, den Inhalator 1 in einen auslösebereiten Zustand zu versetzen und/oder den Inhalator 1 in einem auslösebereiten Zustand zu lagern, aufzubewahren und/oder zu halten. So kann ein Auslösen bzw. Betätigen des Inhalators 1 nach Entnahme aus der Vorrichtung 23 schnell bzw. unverzüglich erfolgen. Die Vorrichtung 23 ist vorzugsweise nicht zum Auslösen bzw. nicht zu einer Betätigung der Auslösetaste 8a ausgebildet.

Alternativ oder zusätzlich kann die Vorrichtung 23 dazu ausgebildet sein, ein Auslösen des Inhalators 1 zu verhindern und/oder zu blockieren, beispielsweise dadurch, dass die Auslösetaste 8a von einem Teil oder Abschnitt der Vorrichtung 23 verdeckt wird. Dies ist jedoch nicht zwingend.

Erfindungsgemäß ist der Inhalator 1 auslösbar ist bzw. die Auslösetaste 8a des Inhalators 1 betätigbar ist, insbesondere manuell und/oder durch einen Benutzer bzw. Patienten, wenn der Inhalator 1 in der Vorrichtung 23 angeordnet bzw. in diese eingesetzt ist. Die Möglichkeit, den Inhalator 1 auszulösen, während er in der Vorrichtung 23 angeordnet bzw. in diese eingesetzt ist, ist vorteilhaft, wenn die Vorrichtung 23 derart ausgebildet ist, dass sie von einem Benutzer bzw. Patienten manuell, insbesondere mit (nur) einer Hand, bewegt bzw. hochgehoben werden kann. In diesem Fall ist es erfindungsgemäß denkbar, dass der Inhalator 1 zur Benutzung nicht aus der Vorrichtung 23 entnommen wird, sondern dass der Inhalator 1 gemeinsam mit der Vorrichtung 23 genommen und zur Inhalation zum Mund des Patienten geführt wird. Vorzugsweise kann dies auch einhändig erfolgen.

Die Fig. 4 und 6 zeigen die Vorrichtung 23 mit dem darin eingesetzten Inhalator 1.

Die Vorrichtung 23 ist vorzugsweise, insbesondere mechanisch und/oder elektronisch, mit dem Inhalator 1 koppelbar oder verbindbar, insbesondere so dass der Inhalator 1 von der Vorrichtung 23 gehalten ist und/oder ein Zustand bzw. Spannzustand des Inhalators 1 bzw. Energiespeichers 7 mittels der Vorrichtung 23 veränderbar ist.

Vorzugsweise ist der Inhalator 1 durch Einsetzen oder Aufstecken mit der Vorrichtung 23 (mechanisch und/oder elektronisch) koppelbar.

Vorzugsweise erfolgt das Koppeln bzw. Einsetzen oder Aufstecken des Inhalators 1 durch eine, insbesondere ausschließlich, geradlinige und/oder axiale Bewegung. Das Koppeln bzw. Einsetzen oder Aufstecken des Inhalators 1 erfolgt vorzugsweise manuell bzw. durch einen Patienten oder Benutzer des Inhalators 1. Grundsätzlich sind hier jedoch auch andere Lösungen denkbar.

Die Vorrichtung 23 weist vorzugsweise eine Standfläche 24 auf, die zumindest im Wesentlichen flach bzw. eben ausgebildet sein kann. In einer Gebrauchslage der Vorrichtung 23 ist die Standfläche 24 auf der Unterseite der Vorrichtung 23 angeordnet oder bildet die Unterseite der Vorrichtung 23 die Standfläche 24.

Relative Orts- bzw. Positionsangaben wie "unten", "oben" o. dgl. beziehen sich jeweils auf die übliche Gebrauchslage der Vorrichtung 23 und/oder des in die Vorrichtung 23 eingesetzten Inhalators 1, bei der die Vorrichtung 23 mit ihrer Standfläche 24 auf einer vorzugsweise im Wesentlichen ebenen und/oder horizontalen Fläche steht bzw. auf dieser aufliegt und der Inhalator 1 dementsprechend mit seiner Drehachse D vertikal ausgerichtet ist, wie auch in den Figuren dargestellt ist. Somit haben die Begriffe "oben", "unten" o. dgl. die übliche Bedeutung bezüglich einer vertikalen bzw. lotrechten Anordnung, wie insbesondere in Fig. 6 dargestellt. "Oben" ist folglich vorzugsweise eine dem Erdmittelpunkt abgewandte Seite, während "unten" eine dem Erdmittelpunkt zugewandte, vorzugsweise quer oder senkrecht zur Gravitationskraft verlaufende Seite ist.

Die Vorrichtung 23 weist vorzugsweise eine Aufnahme 25 für den Inhalator 1 auf oder bildet diese. Insbesondere ist die Aufnahme 25 zum Aufnehmen bzw. zum Einsetzen des Inhalators 1 ausgebildet bzw. ist der Inhalator 1 in die Aufnahme 25 einsetzbar. Die Aufnahme 25 ist dementsprechend korrespondierend zu dem Inhalator 1 geformt.

Die Aufnahme 25 ist insbesondere als Vertiefung und/oder sacklochartige Ausnehmung der Vorrichtung 23 ausgebildet. Die Aufnahme 25 weist vorzugsweise einen Boden 25a an einer axialen Endseite auf. Der Boden 25a verläuft vorzugsweise zumindest im Wesentlichen parallel zu der Standfläche 24 und/oder senkrecht zur Drehachse D.

Die Aufnahme 25 ist vorzugsweise korrespondierend und/oder komplementär zum Inhalator 1 geformt. Die Aufnahme 25 ist vorzugsweise zumindest im Wesentlichen hohlzylindrisch ausgebildet und/oder in radialer Richtung von einer zumindest im Wesentlichen zylindrischen Wandung begrenzt. Die Wandung der Aufnahme 25 ist in den Fig. 4 und 5 nicht gezeigt und in Fig. 6 nur schematisch angedeutet. Die Aufnahme 25 bzw. die die Aufnahme 25 begrenzende Wandung kann einstückig oder mehrteilig ausgebildet sein.

Die Tiefe der Aufnahme 25, also ihre Erstreckung parallel zur Drehachse D, insbesondere gemessen vom Boden 25a bis zum oberen Rand 33, beträgt vorzugsweise mehrere Zentimeter, insbesondere mindestens 3 cm, besonders bevorzugt mindestens 5 cm und/oder höchstens 10 cm, besonders bevorzugt höchstens 7 cm. Dies ist einem sicheren Halten bzw. einer sicheren Lagerung des Inhalators 1 in der Vorrichtung 23 zuträglich.

Vorzugsweise weist die Aufnahme 25 senkrecht zur Drehachse D zumindest im Wesentlichen einen rundlichen, insbesondere ovalen und/oder kreisförmigen, Querschnitt auf. Der Durchmesser der Aufnahme 25 senkrecht zur Drehachse D beträgt vorzugsweise zwischen 2 cm und 5 cm, bevorzugt etwa 3 cm.

Der in die Aufnahme 25 eingesetzte Inhalator 1 bzw. dessen Gehäuseteil 18 und/oder Innenteil 17 kann/können teilweise oder vollständig in der Aufnahme 25 aufgenommen bzw. angeordnet sein oder werden.

Vorzugsweise ist die Aufnahme 25 derart angeordnet, dass die Drehachse D des in die Vorrichtung 23 bzw. Aufnahme 25 eingesetzten Inhalators 1 vertikal verläuft bzw. angeordnet ist, wenn die Standfläche 24 horizontal ausgerichtet ist.

Die Drehachse D des in die Vorrichtung 23 bzw. Aufnahme 25 eingesetzten Inhalators 1 verläuft vorzugsweise senkrecht zur Standfläche 24. Die Aufnahme 25 verläuft mit ihrer Längserstreckung vorzugsweise parallel zur Drehachse D und/oder umgibt die Drehachse D.

Die Drehachse D des in die Vorrichtung 23 eingesetzten Inhalators 1 ist bzw. bildet vorzugsweise (zugleich) eine Mittelachse, Längsachse und/oder Symmetrieachse der Vorrichtung 23 und/oder der Aufnahme 25.

Der Inhalator 1 ist von der Vorrichtung 23 abnehmbar bzw. aus der Aufnahme 25 entnehmbar, insbesondere nach einem Hinzufügen von Energie zu dem Energiespeicher 7 und/oder wenn der Inhalator 1 sich in einem auslösebereiten Zustand befindet. Insbesondere ist der Inhalator 1 durch Entnahme von der Vorrichtung 23 bzw. der Aufnahme 25 entkoppelbar. Es ist möglich, dass die Vorrichtung 23 dazu ausgebildet ist, dass der Inhalator 1 während des Hinzufügens von Energie bzw. Spannens bzw. ungespannt nicht oder nur durch oder nach Entriegelung aus der Vorrichtung 23 entnehmbar ist.

Das Entkoppeln bzw. Abnehmen oder Entnehmen des Inhalators 1 erfolgt vorzugsweise durch eine (ausschließlich) geradlinige und/oder axiale Bewegung. Beispielsweise kann an dem Inhalator 1 und der Vorrichtung 23 eine Nutenführung vorgesehen sein, so dass der Inhalator 1 durch eine (ausschließlich) axiale Bewegung mit der Vorrichtung 23 koppelbar und/oder entkoppelbar ist. Insbesondere weisen der Inhalator 1 und die Vorrichtung 23 dazu zueinander korrespondierende Formschlusselemente, beispielsweise Nuten und Fasen, auf. Vorzugsweise ist durch die Nutenführung eine formschlüssige Kopplung des Inhalators 1 mit der Vorrichtung 23 realisierbar. Insbesondere kann auf diese Weise eine drehfeste Kopplung wenigstens eines der Gehäuseteile 16, 17, 18 des Inhalators 1 mit der Vorrichtung 23 erfolgen. Hierauf wird später noch genauer eingegangen.

Bei der in den Fig. 4 und 5 gezeigten Ausführungsformen der Vorrichtung 23 ist vorzugsweise vorgesehen, dass der Inhalator 1 mit dem Gehäuseunterteil bzw. unteren Gehäuseteil 18 in die Vorrichtung 23 eingesetzt wird. Es sind jedoch auch Ausführungsformen möglich, bei denen der Inhalator 1 ohne das Gehäuseteil 18 in die Vorrichtung 23 einsetzbar ist bzw. eingesetzt wird.

Insbesondere ist bei der in den Fig. 1 bis 6 dargestellten Ausführungsform des Inhalators 1 das untere Gehäuseteil 18 nicht zwingend zum Spannen des Energiespeichers 7 bzw. der Feder erforderlich, obgleich ein manuelles Spannen durch das untere Gehäuseteil 18 erheblich erleichtert wird.

Bei dem beschriebenen Inhalator 1 sind das Gehäuseteil 18 und das Innenteil 17 vorzugsweise derart miteinander verbunden bzw. gekoppelt, dass bei einer Drehung eines der Gehäuseteile 17, 18 das andere Gehäuseteil 17, 18 gleichzeitig und/oder in gleicher Weise gedreht wird. Das Gehäuseteil 17 und das Gehäuseteil 18 sind also drehfest miteinander verbunden bzw. gekoppelt.

Erfindungsgemäß weist die Vorrichtung 23 einen motorischen Antrieb 26 auf. Der Antrieb 26 ist erfindungsgemäß dazu ausgebildet, zwei Gehäuseteile, nämlich die Gehäuseteile 16 und 17 und/oder die Gehäuseteile 16 und 18, des in die Vorrichtung 23 eingesetzten und/oder mit der Vorrichtung 23 gekoppelten Inhalators 1 relativ zueinander zu bewegen, und erfindungsgemäß relativ zueinander zu verdrehen.

Im Folgenden ist der Ausdruck "die Gehäuseteile 16, 17, 18" als Kurzform für "die Gehäuseteile 16 und 17 und/oder die Gehäuseteile 16 und 18" zu verstehen. Analog ist "Gehäuseteil 17, 18" als Kurzform für "Gehäuseteil 17 und/oder Gehäuseteil 18" zu verstehen.

Es ist jedoch auch möglich, dass mittels der Vorrichtung 23 zwei Gehäuseteile des Inhalators 1 anders als drehend zueinander bewegt werden, beispielsweise axial und/oder linear. Vorzugsweise ist jeweils durch die Bewegung der Gehäuseteile 16, 17, 18 zueinander dem Energiespeicher 7 des Inhalators 1 Energie hinzufügbar spannbar bzw. die Feder des Inhalators 1 spannbar. Insbesondere wird durch die Bewegung der Gehäuseteile 16, 17, 18 zueinander die Feder gespannt, insbesondere zusammengedrückt.

Erfindungsgemäß erfolgt das Versetzen des Inhalators 1 in den auslösebereiten Zustand mittels der Vorrichtung 23 also dadurch, dass die Gehäuseteile 16, 17, 18 des in die Vorrichtung 23 eingesetzten Inhalators 1 relativ zueinander bewegt, erfindungsgemäß gegeneinander gedreht, werden, wobei die Feder gespannt bzw. zusammengedrückt wird.

Der Antrieb 26 weist einen erfindungsgemäß elektrischen Motor 27 und/oder eine Übersetzung bzw. ein Getriebe 28 auf. Der Motor 27 ist also vorzugsweise als Elektromotor ausgebildet bzw. elektrisch betreibbar. Vorzugsweise erfolgt mittels des Antriebs 26 ein motorisches und/oder elektrisches Drehen der Gehäuseteile 16, 17 gegeneinander und/oder Hinzufügen von Energie zu dem Energiespeicher 7 bzw. ein Spannen des Inhalators 1. Es ist auch möglich, dass der Antrieb 26 und/oder Motor 27 als Direktantrieb ausgebildet sind.

Der Motor 27 ist in den Figuren nur schematisch dargestellt. Vorzugsweise ist der Motor 27 ein Servomotor und/oder ein Gleichstrommotor, zum Bespiel ein 6 V-Gleichstrommotor. Vorzugsweise beträgt die Stromaufnahme des Motors 27 etwa 0,7 A.

Der Motor 27 kann aber auch ein Asynchronmotor, ein Synchronmotor, ein Wanderwellenmotor und/oder ein Schrittmotor sein.

Der Motor 27 kann auch als Schrittmotor ohne Getriebeunterstützung ausgebildet sein, beispielsweise als Permanentmagnetschrittmotor bzw. High-Torque-Schrittmotor.

Der Antrieb 26 bzw. Motor 27 ist vorzugsweise mit einer Batterie und/oder einem Akku betreibbar. Diese/dieser ist vorzugsweise in die Vorrichtung integriert. Hierauf wird später noch genauer eingegangen.

Die Übersetzung bzw. das Getriebe 28 ist vorzugsweise dazu ausgebildet, eine Bewegung des Motors 27 auf den Inhalator 1 zu übertragen und/oder mittels des Motors 27 eines der Gehäuseteile 16, 17, 18 des Inhalators 1 zu bewegen, insbesondere zu drehen. Mit anderen Worten werden durch die Bewegung jedenfalls eines der Gehäuseteile 16, 17, 18 die beiden Gehäuseteile 16, 17, 18 relativ zueinander bewegt, insbesondere verdreht.

Der Antrieb 26 bzw. Motor 27 ist vorzugsweise dazu ausgebildet, den Energiespeicher 7 bzw. die Feder mit einer Kraft von mindestens 20 N, bevorzugt mindestens 30 N, und/oder höchstens 100 N, bevorzugt höchstens 80 N, besonders bevorzugt höchstens 60 N, zu spannen bzw. zusammenzudrücken. Vorzugsweise wird die Länge des Energiespeichers 7 bzw. der Feder beim Spannen um mindestens 2 mm, bevorzugt mindestens 9 mm, und/oder höchstens 30 mm, bevorzugt höchstens 20 mm, verkürzt.

Die zum Hinzufügen von Energie zu dem Energiespeicher 7 bzw. für einen Spannvorgang aufzuwendende Arbeit oder Energie beträgt vorzugsweise mehr als 20 cNm und/oder weniger als 100 cNm, bevorzugt weniger als 80 cNm, besonders bevorzugt weniger als 60 cNm, insbesondere weniger als 50 cNm. Die Vorrichtung 23, insbesondere der Antrieb 26 und/oder Motor 27, stellt vorzugsweise eine solche Energie und/oder ein solches Drehmoment bereit und/oder verrichtet zum Hinzufügen von Energie zu dem Energiespeicher 7 eine solche Arbeit am Inhalator 1, insbesondere durch bzw. beim Verdrehen der Gehäuseteile 16, 17, 18 zueinander.

Zur Übertragung einer Bewegung von dem Motor 27 auf den Inhalator 1 bzw. mindestens ein Gehäuseteil 16, 17, 18 des Inhalators 1 weist das Getriebe 28 vorzugsweise ein Zahnrad 29a, 29b oder mehrere, vorzugsweise ineinander greifende und/oder zusammenwirkende Zahnräder 29a, 29b auf. Zur Unterscheidung werden die Zahnräder im Folgenden als "erstes" Zahnrad 29a und "zweites" Zahnrad 29b bezeichnet. Es ist auch möglich, dass das "erste" Zahnrad 29a entfällt, so dass nur ein Zahnrad 29a, 29b vorhanden ist, welches aber trotzdem weiterhin als "zweites" Zahnrad 29b bezeichnet wird. Insbesondere impliziert der Ausdruck "zweites Zahnrad" also nicht, dass mehrere Zahnräder vorhanden sein müssen.

Alternativ oder zusätzlich kann das Getriebe 28 ein (nicht dargestelltes) MalteserKreuz-Getriebe, ein Umlaufrädergetriebe, ein Gleitkeilgetriebe, ein Planetengetriebe oder ein sonstiges Getriebe sein.

Der Antrieb 26, der Motor 27 und/oder das Getriebe 28 sind vorzugsweise zumindest teilweise innerhalb der Vorrichtung 23 angeordnet, insbesondere so dass sie für einen (nicht dargestellten) Benutzer verdeckt bzw. nicht sichtbar sind. Vorzugsweise weist die Vorrichtung 23 ein Gehäuse für den Antrieb 26, den Motor 27 und/oder das Getriebe 28 auf.

Vorzugsweise ist mittels des Antriebs 26, des Motors 27 und/oder des Getriebes 28 ein Drehmoment von mindestens 0,2 Nm, bevorzugt mindestens 0,3 Nm, insbesondere mindestens 0,4 Nm, und/oder höchstens 1,1 Nm, bevorzugt höchstens 1,0 Nm, insbesondere höchstens 0,9 Nm, besonders bevorzugt höchstens 0,8 Nm, bereitstellbar und/oder auf den Inhalator 1 bzw. ein Gehäuseteil des Inhalators 1 übertragbar.

Die Übertragung des Drehmoments vom Antrieb 26, Motor 27 und/oder Getriebe 28 auf den Inhalator 1 bzw. ein Gehäuseteil des Inhalators 1 erfolgt vorzugsweise mittels der Kopplungseinrichtung 30b.

Vorzugsweise sind die Vorrichtung 23 und/oder der Antrieb 26 dazu ausgebildet, den Inhalator 1 in höchstens 5 s, bevorzugt höchstens 3 s, insbesondere höchstens 1 s, in den auslösebereiten Zustand zu versetzen bzw. dem Energiespeicher 7 die benötigte Energie hinzuzufügen bzw. die Feder des Inhalators 1 zu spannen. Besonders bevorzugt beträgt die Zeit für das Hinzufügen von Energie zu dem Energiespeicher 7 etwa 0,5 s.

Vorzugsweise weist die Vorrichtung 23 zwei Kopplungseinrichtungen 30a, 30b zum Koppeln des Inhalators 1 und/oder der Gehäuseteile 16, 17, 18 des Inhalators 1 mit der Vorrichtung 23 auf. Es ist jedoch auch möglich, dass die Vorrichtung 23 nur eine oder mehr als zwei Kopplungseinrichtungen 30a, 30b aufweist.

Die Kopplungseinrichtungen 30a, 30b sind vorzugsweise jeweils mit einem von zwei Gehäuseteilen 16, 17 18 des Inhalators 1 insbesondere mechanisch koppelbar, so dass mit den Kopplungseinrichtungen 30a, 30b die zwei Gehäuseteile 16, 17, 18 relativ zueinander bewegbar, insbesondere drehbar, sind. Im konkret dargestellten Ausführungsbeispiel gemäß Fig. 5 und 6 ist beispielsweise die Kopplungseinrichtung 30a mit dem Gehäuseteil 16 und die Kopplungseinrichtung 30b mit dem Gehäuseteil 18 koppelbar.

Vorzugsweise sind die Kopplungseinrichtungen 30a, 30b unterschiedlich ausgebildet.

Vorzugsweise weist die Aufnahme 25 die Kopplungseinrichtung 30a auf oder bildet diese und/oder weist der Antrieb 26 die Kopplungseinrichtung 30b auf oder bildet diese.

Die Vorrichtung 23 ist insbesondere mittels der Kopplungseinrichtungen 30a, 30b mechanisch mit dem Inhalator 1 koppelbar oder (temporär) verbindbar. Insbesondere ist mittels der Kopplungseinrichtungen 30a, 30b eine formschlüssige und/oder drehfeste Verbindung zwischen der Vorrichtung 23 und dem Inhalator 1 bzw. dessen Gehäuseteilen 16, 17, 18 herstellbar.

Bevorzugt greifen also die Kopplungseinrichtungen 30a, 30b und der Inhalator 1 bzw. dessen Gehäuseteile 16, 17, 18 derart formschlüssig ineinander, dass eine relative Bewegung, insbesondere eine Drehung, der Gehäuseteile 16, 17, 18 zueinander ermöglicht ist und/oder dass der Inhalator 1 jeweils durch eine geradlinige und/oder axiale Bewegung mit der Vorrichtung 23 bzw. den Kopplungseinrichtungen 30a, 30b koppelbar und/oder von dieser/diesen entkoppelbar ist.

Vorzugsweise sind die Kopplungseinrichtungen 30a, 30b korrespondierend zu der Form des Inhalators 1 bzw. der Gehäuseteile 16, 17, 18 ausgebildet.

Der Inhalator 1 bzw. das Gehäuseteil 16 und/oder das Gehäuseteil 17, 18 weisen vorzugsweise einen oder mehrere Kopplungsabschnitte 31 auf, die insbesondere als außenseitige und/oder radial vorstehende Erhebungen ausgebildet sind. Dies ist insbesondere in Fig. 3 erkennbar. Besonders bevorzugt weist der Inhalator 1 zwei Kopplungsabschnitte 31 auf.

Ein Kopplungsabschnitt 31 ist vorzugsweise ein Teil oder Abschnitt, der dazu ausgebildet ist, durch Betätigung dem Energiespeicher 7 Energie hinzuzufügen bzw. die Feder zu spannen. Insbesondere handelt es sich um einen oder mehrere Abschnitte, über die Gehäuseteile 16, 17, 18 mechanisch gekoppelt und/oder bewegt werden können, sodass die Feder spannbar ist. Die Kopplungsabschnitte 31 sind vorzugsweise zu der Kopplungseinrichtung 30a, 30b korrespondierend bzw. komplementär gebildet, sodass der Inhalator 1 mittels der Vorrichtung 23 spannbar ist.

Die Kopplungsabschnitte 31 bilden vorzugsweise Vorsprünge und/oder Hinterschnitte derart, dass eine drehsichere bzw. drehfeste Kopplung mit der Vorrichtung 23 ermöglicht wird. Auf diese Weise kann die Vorrichtung 23 die Gehäuseteile 16, 17, 18 relativ zueinander bewegen, insbesondere drehen, sodass die Feder gespannt wird.

Vorzugsweise sind die Kopplungsabschnitte 31 radial und/oder diametral gegenüberliegend und/oder symmetrisch an dem Inhalator 1 bzw. dem Gehäuseteil 16, dem Gehäuseteil 17 und/oder dem Gehäuseteil 18 angeordnet.

Die Kopplungsabschnitte 31 verlaufen in Richtung ihrer Haupterstreckung vorzugsweise axial bzw. parallel zur Drehachse D und/oder sind vorzugsweise länglich ausgebildet.

Vorzugsweise ist durch die Kopplungsabschnitte 31 der Außenumfang des Inhalators 1 bzw. entsprechenden Gehäuseteils 16, 17, 18 im Bereich der Kopplungsabschnitte 31 vergrößert bzw. weist der Inhalator 1 im Bereich der Kopplungsabschnitte 31 einen (aufgrund der Kopplungsabschnitte 31) vergrößerten Umfang auf.

Besonders bevorzugt ist der Inhalator 1 bzw. das Gehäuseteil 16, 17, 18 zumindest im Bereich der Kopplungsabschnitte 31 zumindest im Wesentlichen zylindrisch ausgebildet und/oder weist in diesem Bereich einen zumindest im Wesentlichen kreisförmigen Außenumfang auf, wobei die Kopplungsabschnitte 31 von dem kreis- bzw. zylinderförmigen Abschnitt radial nach außen vorstehen oder nach innen Einbuchtungen bilden.

Der Kopplungsabschnitt 31 kann sich über die beiden Gehäuseteile 16, 17, 18 erstrecken, insbesondere so dass bei aufgestecktem Gehäuseteil 18 insbesondere eine durchgängige Erhebung bzw. Vertiefung gebildet ist.

Durch die Kopplungsabschnitte 31 sind vorzugsweise Angriffsflächen bzw. Widerstände gebildet, durch die das (manuelle) Drehen der Gehäuseteile 16, 17, 18 zueinander für einen nicht dargestellten Benutzer erleichtert ist. Insbesondere erleichtern und/oder ermöglichen die Kopplungsabschnitte 31 bzw. Erhebungen ein relatives Bewegen, insbesondere Verdrehen, der Gehäuseteile 16, 17, 18 zueinander.

Die erste Kopplungseinrichtung 30a und/oder die zweite Kopplungseinrichtung 30b sind vorzugsweise dazu ausgebildet, formschlüssig an den Kopplungsabschnitten 31 bzw. den Erhebungen anzugreifen, diese zu hintergreifen und/oder in diese einzugreifen, wie beispielhaft in Fig. 6 dargestellt. Insbesondere erfolgt auf diese Weise eine mechanische Kopplung bzw. formschlüssige Verbindung der Vorrichtung 23 bzw. der Kopplungseinrichtungen 30a, 30b mit dem Inhalator 1 bzw. den Gehäuseteilen 16, 17, 18.

Vorzugsweise ist die erste Kopplungseinrichtung 30a durch eine oder mehrere Aussparungen 32a gebildet oder weist diese auf.

Die Aussparungen 32a sind im Darstellungsbeispiel an einem oberen und/oder eingangsseitigen Rand 33 der Aufnahme 25, angeordnet. Die Aussparungen 32a können sich jedoch auch ausgehend vom Rand 33 nach unten in die Aufnahme 25 hinein erstrecken, insbesondere parallel zur Drehachse D.

Besonders bevorzugt entspricht die Anzahl der Aussparungen 32a der Anzahl der Kopplungsabschnitte 31. Ganz besonders bevorzugt weist die erste Kopplungseinrichtung 30a zwei Aussparungen 32a auf bzw. ist die erste Kopplungseinrichtung 30a durch zwei Aussparungen 32a gebildet.

Die Aussparungen 32a und/oder der Rand 33 sind vorzugsweise korrespondierend und/oder komplementär zu dem Kopplungsabschnitt 31 bzw. den Kopplungsabschnitten 31 ausgebildet bzw. geformt. Hierdurch kann der Inhalator mit der Vorrichtung 23 gekoppelt werden und/oder bei dem in die Vorrichtung 23 eingesetztem Inhalator 1 der Kopplungsabschnitt 31 formschlüssig gegen ein Verdrehen um die Drehachse D in den Aussparungen 32a gehalten sein. Alternativ oder zusätzlich kann hierdurch die Innenkontur des Rands 33 der Außenkontur des Inhalators 1 bzw. des Gehäuseteils 16, des Gehäuseteils 17 und/oder des Gehäuseteils 18 entsprechen. Vorzugsweise ist das mit der ersten Kopplungseinrichtung 30a verbundene bzw. gekoppelte Gehäuseteil 16, wenn der Inhalator 1 in die Vorrichtung 23 eingesetzt bzw. mit dieser gekoppelt ist, nur axial bzw. parallel zur Drehachse D bewegbar.

Mittels der Kopplungsabschitte 31, der ersten Kopplungseinrichtung 30a und/oder den Aussparungen 32a erfolgt vorzugsweise ein orientiertes Einsetzen des Inhalators 1 in die Vorrichtung 23, insbesondere ein Einsetzen in einer definierten Rotationslage des Inhalators 1 bezüglich der Drehachse D.

Bei dem in den Figuren gezeigten Darstellungsbeispiel weist der Inhalator 1 Erhebungen auf und weist die Vorrichtung 23 den Erhebungen zugeordnete Aussparungen 32a auf. Hier ist jedoch auch eine umgekehrte Anordnung möglich, so dass der Inhalator 1 Aussparungen aufweist und die Vorrichtung 23 Erhebungen oder Vorsprünge aufweist, die in die Aussparungen des Inhalators 1 eingreifen. Des Weiteren sind auch andere Lösungen möglich, bei denen der Inhalator 1 durch Einsetzen in die Vorrichtung 23 formschlüssig mit der Vorrichtung 23 gekoppelt werden kann.

Beispielsweise ist es möglich, dass die erste Kopplungseinrichtung 30a dazu ausgebildet ist, in Griffhilfen bzw. Abflachungen 16a des Gehäuseteils 16 einzugreifen bzw. an diesen anzugreifen. Vorzugsweise erfolgt eine Kopplung der ersten Kopplungseinrichtung 30a mit den Abflachungen 16a mittels einer Federklemmung, wobei (nicht dargestellte) Kopplungselemente der ersten Kopplungseinrichtung 30a mittels Federn radial nach innen vorgespannt oder nach innen spannbar sind, sodass bei oder nach Einsetzen des Inhalators 1 die Kopplungselemente mittels der Federn an die Abflachungen 16a gedrückt werden und somit das Gehäuseteil 16 drehfest halten. Insbesondere kann so eine form- und/oder reinschlüssige Kopplung realisiert sein.

Vorzugsweise ist die erste Kopplungseinrichtung 30a dazu ausgebildet, das (obere) Gehäuseteil 16 bzw. nur eines der Gehäuseteile 16, 17, 18 mittels der Kopplungsabschnitte 31 drehfest in der Vorrichtung 23 zu halten. Durch das Einsetzen des Inhalators 1 wird vorzugsweise also nur eines der Gehäuseteile 16, 17, 18 mit der Kopplungseinrichtung 30a gekoppelt bzw. formschlüssig mit dieser verbunden. Das andere Gehäuseteil 16, 17, 18, insbesondere das Gehäuseteil 17 und/oder das Gehäuseteil 18, sind vorzugsweise relativ zu der Kopplungseinrichtung 30a bewegbar, insbesondere verdrehbar, wenn der Inhalator 1 in die Vorrichtung 23 eingesetzt bzw. mit dieser gekoppelt ist.

Die zweite Kopplungseinrichtung 30b ist vorzugsweise relativ zu der Aufnahme 25 und/oder relativ zu der ersten Kopplungseinrichtung 30a bewegbar, insbesondere verdrehbar, angeordnet bzw. ausgebildet.

Die zweite Kopplungseinrichtung 30b ist vorzugsweise dazu ausgebildet, eines der Gehäuseteile 16, 17, 18, insbesondere das Innenteil 17 und/oder das untere Gehäuseteil 18, zu bewegen, sodass durch die Bewegung des Gehäuseteils 17, 18 die Gehäuseteile 16, 17, 18 gegeneinander bzw. relativ zueinander gedreht bzw. verdreht werden.

Vorzugsweise ist die zweite Kopplungseinrichtung 30b durch eines der Zahnräder 29a, 29b gebildet und/oder einstückig mit einem der Zahnräder 29a, 29b, insbesondere dem ersten Zahnrad 29a, ausgebildet.

Über die zweite Kopplungseinrichtung 30b ist dem Energiespeicher 7 vorzugsweise mittels des Antriebs 26 Energie hinzufügbar bzw. ist vorzugsweise mittels des Antriebs 26 die Feder spannbar. Insbesondere ist die zweite Kopplungseinrichtung 30b mittels des Motors 27 antreibbar und die zweite Kopplungseinrichtung 30b bei eingesetztem Inhalator 1 derart mit einem der Gehäuseteile 16, 17, 18 des Inhalators 1 gekoppelt, dass eine Bewegung des Motors 27 die Gehäuseteile 16, 17, 18 derart zueinander bewegt, insbesondere verdreht, dass dem Energiespeicher 7 Energie hinzugefügt bzw. die Feder gespannt wird.

Besonders bevorzugt bildet die zweite Kopplungseinrichtung 30b ein Zahnrad des Getriebes 28. Hierbei kann die zweite Kopplungseinrichtung 30b radial innenseitig zum formschlüssigen Greifen des Gehäuseteils 16, 17, 18 und radial außenseitig mit Zähnen zur Bildung des ersten Zahnrads 29a ausgebildet sein. Mit anderen Worten weist der Antrieb 26, insbesondere das Getriebe 28, ein Zahnrad 29a auf, das durch den Motor 27 und/oder das zweite Zahnrad 29b antreibbar ist und innenseitig die zweite Kopplungseinrichtung 30b aufweist. Das zweite Zahnrad 29b kann entfallen, so dass der Antrieb 26 und/oder Motor 27 das erste Zahnrad 29a direkt antreibt.

Die zweite Kopplungseinrichtung 30b bzw. das die zweite Kopplungseinrichtung 30b bildende oder aufweisende erste Zahnrad 29a ist vorzugsweise am Boden 25a der insbesondere sacklochartig ausgebildeten Aufnahme 25 und/oder auf einer dem Rand 33 abgewandten Seite der Aufnahme 25 angeordnet. Insbesondere ist die zweite Kopplungseinrichtung 30b unterhalb der ersten Kopplungseinrichtung 30a und/oder dem Rand 33 gegenüberliegend angeordnet. Vorzugsweise ist die zweite Kopplungseinrichtung 30b an einer axialen Endseite und/oder innerhalb der Aufnahme 25 angeordnet, wie in Fig. 6 schematisch angedeutet.

Die zweite Kopplungseinrichtung 30b bzw. das die Kopplungseinrichtung 30b bildende oder aufweisende erste Zahnrad 29a ist vorzugsweise derart in der Vorrichtung 23 bzw. der Aufnahme 25 angeordnet, dass die Haupterstreckungsebene des erste Zahnrads 29a quer, insbesondere senkrecht, zu der Drehachse D verläuft und/oder dass Zähne 34 des erste Zahnrads 29a bzgl. der Drehachse D radial von dem erste Zahnrad 29a abragen.

Das erste Zahnrad 29a ist vorzugsweise symmetrisch und/oder derart zu der Drehachse D angeordnet, dass die Drehachse D auch die Rotationsachse des ersten Zahnrads 29a bildet.

Vorzugsweise ist die zweite Kopplungseinrichtung 30b bzw. das die zweite Kopplungseinrichtung 30b bildende oder aufweisende erste Zahnrad 29a zumindest teilweise hohlzylindrisch und/oder ringartig ausgebildet, insbesondere wobei die Zähne 34 nach innen und/oder außen radial von dem Ring bzw. Hohlzylinder abragen. Die Zähne 34 erstrecken sich in axialer Richtung vorzugsweise nur über einen Teil des ersten Zahnrads 29a bzw. Hohlzylinders bzw. Rings, sodass, insbesondere an einem oberen Ende des ersten Zahnrads 29a, ein kragenartiger oder kranzartiger, vorzugsweise (hohl-)zylindrischer Abschnitt ohne Zähne 34 bzw. ein zahnloser Abschnitt gebildet ist.

Vorzugsweise umgibt die Kopplungseinrichtung 30b bzw. das erste Zahnrad 29a den in die Vorrichtung 23 eingesetzten Inhalator 1 bzw. dessen Gehäuseteil 17 und/oder Gehäuseteil 18 zumindest teilweise. Der in die Vorrichtung 23 eingesetzte Inhalator 1 bzw. dessen Gehäuseteil 17, 18 ist vorzugsweise zumindest teilweise radial innerhalb und/oder auf der Innenseite der Kopplungseinrichtung 30b angeordnet. Insbesondere umfasst oder umschließt die zweite Kopplungseinrichtung 30b einen unteren Abschnitt des in die Vorrichtung 23 eingesetzten Inhalators 1 bzw. des Gehäuseteils 17, 18.

Vorzugsweise weist die zweite Kopplungseinrichtung 30b bzw. der zahnlose Abschnitt eine oder mehrere, besonders bevorzugt zwei, Aussparungen 32b zur Kopplung des ersten Zahnrads 29a bzw. der zweiten Kopplungseinrichtung 30b mit dem Kopplungsabschnitt 31 bzw. den Kopplungsabschnitten 31 auf.

Im dargestellten Ausführungsbeispiel sind die Aussparungen 32b an einem oberen Rand des ersten Zahnrads 29a bzw. der Kopplungseinrichtung 30b angeordnet und/oder rechteckig ausgebildet. Vorzugsweise liegen sich die Aussparungen 32b radial bzw. diametral gegenüber.

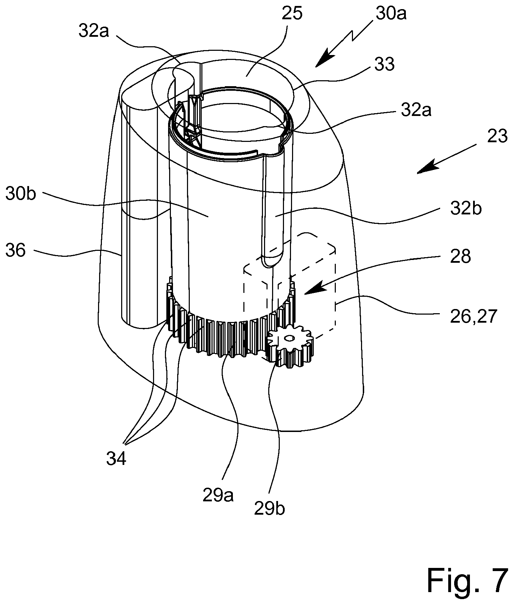

Gemäß einer weiteren, in Fig. 7 dargestellten Ausführungsform ist die Kopplungseinrichtung 30b nach Art eines Bechers mit einem Becherboden und einer zumindest im Wesentlichen zylindrischen Seitenfläche ausgebildet. Dieser Becher kann einstückig mit dem ersten Zahnrad 29a ausgebildet und/oder an dem ersten Zahnrad 29a angeformt sein und/oder auf andere Weise drehfest mit dem ersten Zahnrad 29a gekoppelt bzw. verbunden sein. Im Darstellungsbeispiel weist der Becher außen an einem unteren axialen Ende bzw. becherbodenseitigen Abschnitt der Seitenfläche die Zähne 34 auf. Vorzugsweise sind bei dem Becher die Aussparungen 32b in der Seitenfläche, insbesondere dem zahnlosen Abschnitt, ausgebildet, sodass die Kopplungsabschnitte 31 drehfest in die Aussparungen 32b eingreifen und/oder mit diesen gekoppelt sind, wenn der Inhalator 1 in die Aufnahme 25 bzw. den Becher eingesetzt ist. Insbesondere ist der Becher derart ausgebildet, dass das Gehäuseteil 17, 18 zumindest großflächig und/oder vollständig an der Seitenfläche des Bechers oder den Aussparungen 32b, anliegt, wenn der Inhalator 1 bzw. das Gehäuseteil 17, 18 in den Becher eingesetzt ist. Vorzugsweise ist das erste Zahnrad 29a unterhalb bzw. an einem unteren axialen Ende des Bechers angeordnet, wenn es nicht einstückig mit dem Becher ausgebildet ist bzw. der Becher selbst keine Zähne 34 aufweist. Hier sind jedoch auch andere Lösungen möglich.

Der Inhalator 1 ist vorzugsweise durch axiales und/oder geradliniges Einsetzen oder Einschieben mit der zweiten Kopplungseinrichtung 30b, insbesondere den Aussparungen 32b, verbindbar bzw. koppelbar oder in dieser einsetzbar, vorzugsweise wobei die Kopplungsabschnitte 31 beim Einsetzen bzw. des eingesetzten Inhalators 1 in die Aussparungen 32b eingreifen und auf diese Weise das entsprechende Gehäuseteil 17, 18 drehfest mit der zweiten Kopplungseinrichtung 30b verbinden bzw. koppeln.

Der (in die Vorrichtung 23 eingesetzte) Inhalator 1 ist vorzugsweise axial zur zweiten Kopplungseinrichtung 30b bewegbar, insbesondere von dieser entkoppelbar bzw. abnehmbar oder entnehmbar und/oder drehfest, insbesondere formschlüssig, mit der zweiten Kopplungseinrichtung 30b gekoppelt, bevorzugt mittels der Aussparungen 32b.

Die Kopplungseinrichtung 30a bzw. 30b kann aber auch derart ausgebildet sein, dass sie keine Aussparungen aufweist. Insbesondere ist es möglich, dass die Kopplungseinrichtung 30b den Inhalator 1 bzw. eines der Gehäuseteile 16, 17, 18 greift, umgreift und/oder in eines der Gehäuseteile 16, 17, 18 eingreift.

Generell ist vorgesehen, dass die Kopplungseinrichtung 30a, 30b form- und oder reibschlüssig, drehfest mit dem Inhalator bzw. einem der Gehäuseteile 16, 17, 18 verbindbar ist.

Vorzugsweise ist durch die zweite Kopplungseinrichtung 30b und/oder Aussparungen 32b eine axiale Endposition für den Inhalator 1 definiert. Der Inhalator 1 ist, insbesondere aufgrund der Kopplungsabschnitte 31, also vorzugsweise nicht weiter als bis in die durch die Aussparungen 32b definierte Endposition in die Aufnahme 25 bzw. die Vorrichtung 23 bewegbar, insbesondere einschiebbar oder aufsteckbar.

Durch die Aussparungen 32b und/oder die Kopplungsabschnitte 31 ist also vorzugsweise die Bewegung des Inhalators 1 nach unten bzw. in Richtung des Bodens 25a der Aufnahme 25 begrenzt. Dies ermöglicht ein einfaches und definiertes Einsetzen des Inhalators 1 in die Vorrichtung 23.

Die Aufnahme 25 ist vorzugsweise zum Halten des Inhalators 1 und/oder zumindest eines der Gehäuseteile 16, 17, 18 ausgebildet.

Vorzugsweise wird beim und/oder durch Einsetzen des Inhalators 1 in die Aufnahme 25 bzw. die Vorrichtung 23 der Antrieb 26 mit einem der Gehäuseteile 16, 17, 18, insbesondere dem Innenteil 17 und/oder dem unteren Gehäuseteil 18, verbunden und das andere Gehäuseteil 16, 17, 18, insbesondere das (obere) Gehäuseteil 16, mit der Aufnahme 25, insbesondere der Kopplungseinrichtung 30a bzw. den Aussparungen 32a, verbunden. Die Verbindung des Antriebs 26 mit einem der Gehäuseteile 16, 17, 18 erfolgt vorzugsweise mittels der zweiten Kopplungseinrichtung 30b und/oder der Aussparungen 32b.

Vorzugsweise sind die genannten Verbindungen derart ausgestaltet, dass das mit dem Antrieb 26 (drehfest) verbundene Gehäuseteil 16, 17, 18 relativ zu dem anderen Gehäuseteil 16, 17, 18 bewegbar, insbesondere drehbar, ist und das andere Gehäuseteil 16, 17, 18 zumindest während des Spannens unbeweglich, vorzugsweise drehfest, in der Aufnahme 25 bzw. der ersten Kopplungseinrichtung 30a gehalten ist.

Zum Hinzufügen von Energie zu dem Energiespeicher 7 bzw. zum Versetzen des Inhalators 1 in den auslösebereiten Zustand wird vorzugsweise eines der Zahnräder 29a, 29b mittels des Antriebs 26 bzw. Motors 27 angetrieben, insbesondere in Rotation versetzt. Das vom Antrieb 26 bzw. Motor 27 angetriebene zweite Zahnrad 29b greift vorzugsweise in die Kopplungseinrichtung 30b bzw. das erste Zahnrad 29a mit der Kopplungseinrichtung 30b ein und/oder ist damit verzahnt, sodass die Kopplungseinrichtung 30b um die Drehachse D gedreht wird bzw. in Rotation versetzt wird. Wenn der Inhalator 1 in die Vorrichtung 23 eingesetzt ist, werden so das Gehäuseteil 17 und/oder das Gehäuseteil 18 gegen das Gehäuseteil 16 gedreht, wodurch die Feder gespannt wird und der Inhalator 1 somit in den auslösebereiten Zustand versetzt wird.

Prinzipiell ist auch möglich, dass mehr als zwei Zahnräder 29a, 29b verwendet werden oder dass der Antrieb 26 bzw. Motor 27 die Kopplungseinrichtung 30b unmittelbar antreibt bzw. dreht. In diesem Fall kann die Kopplungseinrichtung 30b auch ohne das erste Zahnrad 29a ausgebildet sein, beispielsweise wenn ein Teil des Antriebs 26 bzw. Motors 27 unmittelbar formschlüssig mit der Kopplungseinrichtung 30b verbindbar oder verbunden ist.

In einer nicht dargestellten Alternative weist der Antrieb 26 eine Schnecke auf. Die Schnecke kann unmittelbar von der Motorwelle des Motors 27 angetrieben werden. Die Schnecke greift vorzugsweise unmittelbar in eine hierzu komplementäre, am äußeren radialen Umfang der zweiten Kopplungseinrichtung 30a gebildete Verzahnung ein. Auf diese Weise kann mit einfachen Mitteln eine relativ hohe Untersetzung erzeugt werden, was die Bereitstellung des notwendigen Drehmoments erleichtert.

Grundsätzlich gibt es jedoch auch hiervon abweichende Antriebskonzepte.

Die zweite Kopplungseinrichtung 30b ist allgemein vorzugsweise mittels des Getriebes 28 von dem Antrieb 26 bzw. Motor 27 bewegbar, insbesondere drehbar.

Die Vorrichtung 23 und/oder der Antrieb 26 sind vorzugsweise dazu ausgebildet, die Gehäuseteile 16, 17, 18, insbesondere um mehr als 90° und/oder um höchstens 360°, insbesondere um zumindest im Wesentlichen 180°, gegeneinander zu verdrehen.

Besonders bevorzugt erfolgt das Spannen des Inhalators 1 bzw. Hinzufügen von Energie zu dem Energiespeicher 7 bzw. das Versetzen in den auslösebereiten Zustand durch eine Drehung der Gehäuseteile 16, 17, 18 gegeneinander um 180° bzw. durch eine halbe Umdrehung des Innenteils 17 und/oder unteren Gehäuseteils 18 gegenüber dem Gehäuseteil 16.

Dementsprechend sind die Vorrichtung 23 und/oder der Antrieb 26 vorzugsweise dazu ausgebildet, die Gehäuseteile 16, 17, 18 um 180° gegeneinander zu verdrehen. Es ist aber auch möglich, dass ein Spannen des Inhalators 1 bzw. Hinzufügen von Energie zu dem Energiespeicher 7 mittels einer Drehung um einen anderen Winkel, beispielsweise 90° oder 360°, erfolgt. Zum Versetzen in den auslösebereiten Zustand bzw. zum Spannen der Feder ist jedoch vorzugsweise keine vollständige Drehung nötig.

Insbesondere kann der Inhalator 1 und/oder die Vorrichtung 23 derart ausgebildet sein, dass die Gehäuseteile 16, 17, 18 nur um maximal 180° in einem ununterbrochenen Drehvorgang relativ zueinander verdrehbar sind bzw. dass ein Verdrehen um mehr als 180° in einer einzigen, ununterbrochenen Bewegung blockiert ist.

Vorzugsweise sind die Vorrichtung 23 und/oder der Antrieb 26 dazu ausgebildet, die Gehäuseteile 16, 17, 18 um einen festen (ganzzahligen) Teilwinkel von 360° gegeneinander zu verdrehen, vorzugsweise wobei die Gradzahl des Winkels ein (ganzzahliger) Teiler von 360 ist. Der feste Teilwinkel kann also beispielsweise 180°, 120°, 90°, 60°, 45° oder 30° betragen. Andere Teilwinkel sind jedoch auch möglich.

Die Vorrichtung 23 weist vorzugsweise einen oder mehrere Sensoren 35 zur Detektion des Einsetzens, Koppelns und/oder Zustands bzw. Spannzustands des Inhalators 1 auf. Der Sensor 35 ist in den Figuren nur schematisch dargestellt.

Vorzugsweise ist mittels des Sensors 35 feststellbar, ob der Inhalator 1 vollständig und/oder korrekt in die Vorrichtung 23 eingesetzt wurde, insbesondere so dass der Inhalator 1 mit den Kopplungseinrichtungen 30a, 30b gekoppelt ist und/oder ein Hinzufügen von Energie zu dem Energiespeicher 7, insbesondere durch Drehen der Gehäuseteile 16, 17, 18 gegeneinander, ermöglicht ist.

Der Sensor 35 ist vorzugsweise der Aufnahme 25, der Kopplungseinrichtung 30a und/oder der Kopplungseinrichtung 30b zugeordnet. Der Sensor 35 ist vorzugsweise dazu ausgebildet, ein Einsetzen des Inhalators 1 in die Aufnahme 25 und/oder ein Koppeln des Inhalators 1 bzw. Gehäuseteils 16, 17, 18 des Inhalators 1 mit einer der Kopplungseinrichtungen 30a, 30b oder beiden Kopplungseinrichtungen 30a, 30b zu detektieren. Besonders bevorzugt ist der Sensor 35 dazu ausgebildet, die Positionierung des Inhalators 1 in einer Gebrauchslage zu detektieren, in der der Inhalator 1 mittels der Vorrichtung 23 spannbar bzw. dem Energiespeicher 7 Energie hinzufügbar ist. Diese Lage ist insbesondere dadurch gekennzeichnet, dass die Kopplungseinrichtungen 30a, 30b derart mit den Gehäuseteilen 16, 17, 18 verbunden sind, dass der Antrieb 26 diese zueinander bewegen, insbesondere verdrehen kann. Hierdurch kann der Inhalator 1 gespannt werden. Vorzugsweise ist in dieser Lage der Inhalator 1 vollständig in die Vorrichtung 23 eingeschoben und/oder sind in dieser Lage die Kopplungsabschnitte 31 vollständig in die Aussparungen 32b eingeschoben.

Zur Detektion des Einsetzens und/oder Koppelns des Inhalators 1 kann der Sensor 35 insbesondere einen (Mikro-)Schalter oder Taster aufweisen oder dadurch gebildet sein, der vorzugsweise durch Einsetzen des Inhalators 1 automatisch betätigbar ist bzw. betätigt wird. In diesem Fall ist es bevorzugt, dass der Sensor 35 an der Unterseite bzw. am Boden 25a der Aufnahme 25 angeordnet ist, insbesondere sodass der Schalter oder Taster beim Einsetzen des Inhalators 1 mit diesem in Kontakt kommt und somit betätigt wird. Eine - alternativ oder zusätzlich - seitliche Anordnung des Sensors 35 ist jedoch ebenfalls möglich.

Vorzugsweise ist der Sensor 35 dazu ausgebildet, ein richtiges Einsetzen und/oder Koppeln des Inhalators 1 zu detektieren. Insbesondere ist mittels des Sensors 35 detektierbar, ob der Inhalator 1 derart in die Vorrichtung 23 eingesetzt wurde, das ein Verdrehen der Gehäuseteile 16, 17, 18 gegeneinander und/oder ein Hinzufügen von Energie zu dem Energiespeicher 7 möglich ist.

Die Vorrichtung 23 kann auch einen (weiteren) Sensor 35 aufweisen, mit dem alternativ oder zusätzlich der Zustand bzw. Spannzustand des Inhalators 1 detektierbar ist. Insbesondere wenn ein Einsetzen des Inhalators 1 in die Vorrichtung 23 ohne das Gehäuseteil 18 vorgesehen ist und/oder wenn das untere Gehäuseteil 18 durchsichtig ist, kann der Zustand bzw. Spannzustand des Inhalators 1 über die axiale Position des Behälters 3 detektiert werden, da der Zustand bzw. Spannzustand mit dieser Position vorzugsweise korreliert ist, wie weiter oben beschrieben.

Eine Detektion des Zustandes bzw. Spannzustandes kann dann optisch, beispielsweise über eine Reflexions- und/oder Abstandsmessung erfolgen. Es ist auch möglich, dass der (weitere) Sensor 35 als Schalter oder Taster ausgebildet ist, der vorzugsweise am Boden der Aufnahme 35 angeordnet ist. Mittels eines solchen Sensors 35 wird bei Einsetzen des Inhalators 1 ohne unteres Gehäuseteil 18 abhängig vom Zustand bzw. Spannzustand bzw. der axialen Position des Behälters 3 der Schalter oder Taster betätigt oder nicht betätigt, sodass der Zustand bzw. Spannzustand detektierbar bzw. ermittelbar ist. Vorzugsweise weist die Vorrichtung 23 in diesem Fall zwei Sensoren 35 auf, sodass (zusätzlich) das Einsetzen des Inhalators 1 unabhängig von dessen Zustand bzw. Spannzustand detektierbar ist. Dies ist insbesondere notwendig, wenn der Inhalator 1 sich beim Einsetzen in die Vorrichtung 23 in einem Zustand bzw. Spannzustand befindet, bei dem der Behälter 3 sich in einer axialen Position befindet, in der beim Einsetzen keine Betätigung des zur Detektion des Zustandes bzw. Spannzustandes ausgebildeten Schalters oder Tasters erfolgt.

Es sind jedoch auch andere und/oder mehrere Sensoren 35 denkbar, die beispielsweise das Einsetzen und/oder Koppeln und/oder den Zustand bzw. Spannzustand des Inhalators 1 kapazitiv, induktiv und/oder optisch erfassen. Grundsätzlich ist demzufolge auch eine Ermittlung des Zustandes bzw. Spannzustandes des Inhalators 1 und/oder eine Ermittlung der Position des Behälters 3 möglich, wenn der Inhalator 1 mit dem vorzugsweise transparenten Gehäuseteil 18 in die Vorrichtung eingesetzt ist oder wird.

Zur Detektion des Zustands bzw. Spannzustands des Inhalators 1 bzw. des Energiespeichers 7 kann der Sensor 35 insbesondere als optischer Sensor bzw. zur optischen Detektion ausgebildet sein. Wie weiter oben bereits ausgeführt, korreliert der Zustand bzw. Spannzustand des Energiespeichers 7 mit einer axialen Position des Behälters 3 bzw. lässt die axiale Position des Behälters 3 (eindeutig) auf den Zustand bzw. Spannzustand des Energiespeichers 7 schließen. So ist über eine Detektion des Vorhandenseins des Behälters 3 und/oder der Lage, insbesondere axialen Position, des Behälters 3 mittels des Sensors 35 (indirekt) eine Detektion des Zustandes bzw. Spannzustandes des Energiespeichers 7 bzw. des Inhalators 1 möglich.

Insbesondere kann der Sensor 35 dazu ausgebildet sein, das Vorhandensein des Behälters 3 und/oder die Lage bzw. axiale Position des Behälters 3 mittels einer Reflexionsmessung von am Behälterboden 21 reflektierten Licht zu detektieren, erfassen und/oder bestimmen. Alternativ oder zusätzlich kann der Sensor 35 die Lage des Inhalators 1, des Behälters 3 und/oder den Zustand bzw. Spannzustand kapazitiv, induktiv, durch Detektion einer Berührung oder eines Drucks, auf die beschriebene oder andere Weise optisch oder auf sonstige Weise detektieren.

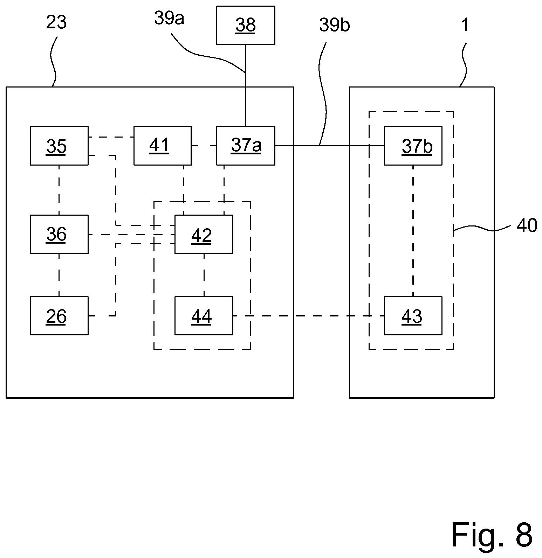

Die Fig. 8 veranschaulicht anhand eines schematischen Blockschaltbilds ein aus dem Inhalator 1 und der Vorrichtung 23 gebildetes System. Gestrichelte Linien deuten dabei insbesondere elektrische und/oder Datenverbindungen zwischen verschiedenen Komponenten bzw. Einrichtungen an.

Vorzugsweise weist die Vorrichtung 23 eine Steuerung 36 auf. Die Steuerung 36 ist vorzugsweise dazu ausgebildet, mit dem Sensor 35 das Einsetzen und/oder Koppeln des Inhalators 1 zu detektieren, vorzugsweise wobei die Steuerung 36 und der Sensor 35 elektrisch und/oder informationstechnisch miteinander verbunden sind, wie in Fig. 8 angedeutet. Vorzugsweise ist die Steuerung 36 ebenfalls dazu ausgebildet, mit dem Sensor 35 oder auf andere Weise oder mit einem anderen Sensor den Zustand bzw. Spannzustand des Inhalators 1 zu detektieren.

Besonders bevorzugt ist die Steuerung 36 dazu ausgebildet, in Abhängigkeit des Zustands bzw. Spannzustands den Antrieb 26 bzw. Motor 27 zu steuern, vorzugsweise sodass der Inhalator 1 automatisch durch Hinzufügen von Energie zu dem Energiespeicher 7 in den auslösebereiten bzw. gespannten Zustand versetzt wird. Vorzugsweise ist die Steuerung 36 mit dem Antrieb 26 elektrisch und/oder informationstechnisch verbunden, wie in Fig. 8 angedeutet.

Vorzugsweise ist die Steuerung 36 dazu ausgebildet, das Drehmoment des Antriebs 26 bzw. Motors 27 zu begrenzen. So kann eine Beschädigung des Inhalators 1 und/oder der Vorrichtung 23, insbesondere des Antriebs 26 bzw. Motors 27 selbst, vermieden werden.

Vorzugsweise wird das vom Antrieb 26 bzw. Motor 27 aufgebrachte Drehmoment von der Steuerung 36 auf einen Höchstwert von 0,5 Nm bis 0,8 Nm begrenzt. Dies ermöglicht auf einfache Weise einen Schutz vor einem "Überdrehen" des Inhalators 1 und/oder einer daraus resultierenden Beschädigung. Insbesondere weist der Inhalator 1 nämlich ein Sperrspannwerk auf, dass vorzugsweise wie in der WO 97/20590 beschrieben ausgebildet ist. Dieses Sperrspannwerk soll verhindern, dass die Gehäuseteile 16, 17, 18 in einer Drehbewegung um mehr als 180° gegeneinander verdreht werden. Diese durch das Sperrspannwerk realisierte Sperre kann jedoch übermäßigem Krafteinsatz überwunden werden, wobei das Sperrspannwerk jedoch abnutzt bzw. beschädigt wird. Eine derartige Abnutzung oder Beschädigung kann durch die Begrenzung des vom Antrieb 26 bzw. Motor 27 aufgebrachten Drehmoments verhindert werden.

Eine Begrenzung des Drehmoments kann bei einem als Elektromotor ausgebildeten Antrieb 26 bzw. Motor 27 durch eine Begrenzung oder Unterbrechung der Stromzufuhr zu dem Antrieb 26 bzw. Motor 27 erfolgen. Insbesondere ist die Steuerung 36 dementsprechend auch dazu ausgebildet, die Stromzufuhr bzw. den Stromverbrauch des Antriebs 26 bzw. Motors 27 festzustellen, insbesondere zu messen, und in Abhängigkeit davon den Antrieb 26 bzw. den Motor 27 bzw. die Stromzufuhr zu steuern oder zu regeln, insbesondere zu begrenzen oder zu unterbrechen.

Mit anderen Worten ist die Vorrichtung 23, die Steuerung 36 und/oder der Antrieb 26 also vorzugsweise dazu ausgebildet, einen Spannvorgang bzw. ein Hinzufügen von Energie zu dem Energiespeicher 7 beim Erreichen eines definierten bzw. vorgegebenen Maximaldrehmoments bzw. einer definierten bzw. vorgegebenen Maximalarbeit zu stoppen, abzubrechen und/oder zu unterbrechen, insbesondere den Antrieb 26 bzw. Motor 27 anzuhalten oder abzuschalten. Das Maximaldrehmoment bzw. die Maximalarbeit beträgt vorzugsweise 0,5 Nm, 0,6 Nm oder 0,8 Nm. Vorzugsweise erfolgt ein Drehen der Gehäuseteile 16, 17, 18 gegeneinander mittels der Vorrichtung 23 nur mit Drehmomenten, die kleiner als das Maximaldrehmoment sind.

Die Steuerung 36 weist vorzugsweise einen Steuerchip, insbesondere einen Mikrocontroller oder einen ASIC, auf. Der Steuerchip weist vorzugsweise ein Eingangs-Interface für Steuergeräte wie Sensoren, insbesondere den Sensor 35, und/oder ein Ausgangs-Interface, insbesondere zur Ansteuerung des Antriebs 26, auf.

Die Steuerung 36 ist vorzugsweise zum bedarfsweisen Hinzufügen von Energie zu dem Energiespeicher 7 ausgebildet.

Insbesondere ist die Steuerung 36 dazu ausgebildet, mittels des Sensors 35 den Zustand bzw. Spannzustand des Energiespeichers 7 zu erkennen bzw. zu erkennen, ob ein Hinzufügen von Energie zu dem Energiespeicher 7 möglich oder erforderlich ist. Insbesondere wird der Antrieb 26 von der Steuerung 36 nur dann eingeschaltet bzw. betrieben oder gesteuert, wenn sich der in die Vorrichtung 23 eingesetzte Inhalator 1 im ungespannten oder nicht auslösebereiten Zustand befindet, wenn also eine Benutzung oder Auslösung des Inhalators 1 zunächst durch Hinzufügen von Energie zu dem Energiespeicher 7 in den auslösebereiten Zustand vorbereitet werden muss. Auf diese Weise werden ein unnötiges Spannen und/oder damit einhergehende oder daraus folgende Beschädigungen des Inhalators 1 und/oder der Vorrichtung 23 vermieden.

Es ist jedoch auch möglich, dass das Hinzufügen von Energie zu dem Energiespeicher 7 nicht automatisch (bei Einsetzen) erfolgt und/oder - alternativ oder zusätzlich - von einem (nicht dargestellten) Benutzer manuell steuerbar oder bewirkbar ist, beispielsweise durch Betätigen eines entsprechenden (in Fig. 3 bis 6 nicht dargestellten) Knopfs oder Schalters an der Vorrichtung 23, wobei die Betätigung des Knopfs oder Schalters ein Hinzufügen von Energie zu dem Energiespeicher 7 mittels des Antriebs 26 bewirkt oder zur Folge hat. Die Auslösung des Spannvorgangs erfolgt auch hierbei vorzugsweise in Abhängigkeit von dem detektierten Zustand bzw. Spannzustand des Inhalators 1. Die Steuerung 36 ist hierbei vorzugsweise dazu ausgebildet, bei Aktivierung, beispielsweise durch eine Auslöseeinrichtung wie einen Knopf oder Schalter, den Spannvorgang, also insbesondere den Antrieb 26, lediglich dann zu starten, wenn sich der Inhalator 1 in seinem ungespannten bzw. nicht auslösebereiten Zustand befindet. Alternativ oder zusätzlich wird die Aktivierung des Antriebs 26 durch die Steuerung 36 blockiert, wenn sich der Inhalator 1 bereits in seinem auslösebereiten Zustand befindet bzw. dem Energiespeicher 7 bereits Energie hinzugefügt wurde bzw. die Feder bereits gespannt ist.

Vorzugsweise kann die Vorrichtung 23 in einem Sleep-Modus (Ruhezustand) mit geringem oder minimiertem Energieverbrauch betrieben werden. Besonders bevorzugt weist die Steuerung 36 eine Aktivierungsschaltung zur Aktivierung der Vorrichtung 23 aus dem Sleep-Modus auf.

Die Steuerung 36 ist vorzugsweise dazu ausgebildet, dass nach einem Hinzufügen von Energie zu dem Energiespeicher 7 der Sleep-Modus automatisch aktiviert wird und/oder das zum Hinzufügen von Energie zu dem Energiespeicher 7 die Vorrichtung 23 aus dem Sleep-Modus aktiviert wird. Insbesondere erfolgt diese Aktivierung durch eine Detektion eines Einsetzens des Inhalators 1 oder durch Betätigen der Taste zum Hinzufügen von Energie zu dem Energiespeicher 7.

Die Vorrichtung 23 weist vorzugsweise eine Kommunikationseinrichtung 37a auf. Die Kommunikationseinrichtung 37a der Vorrichtung 23 kann bedarfsweise mehrteilig ausgebildet sein. Insbesondere ist die Vorrichtung 23 mittels der Kommunikationseinrichtung 37a als Dockingstation (für den Inhalator 1) ausgebildet.

Der Inhalator 1 weist vorzugsweise eine Kommunikationseinrichtung 37b auf. Die Kommunikationseinrichtung 37b des Inhalators 1 kann in dem unteren Gehäuseteil 18 angeordnet sein, insbesondere am Boden des Gehäuseteils 18, wie in Fig. 3 schematisch angedeutet ist.

Die Kommunikationseinrichtung 37a der Vorrichtung 23 ist vorzugsweise zur bevorzugt drahtlosen Kommunikation mit einem externen System 38 und/oder mit dem Inhalator 1 bzw. dessen Kommunikationseinrichtung 37b ausgebildet. Insbesondere kann bei einer mehrteilig ausgebildeten Kommunikationseinrichtung 37a der Vorrichtung 23 ein Teil zur Kommunikation mit dem externen System 38 und ein anderer Teil zur Kommunikation mit dem Inhalator 1 bzw. dessen Kommunikationseinrichtung 37b ausgebildet sein.

Vorzugsweise ist zwischen der Kommunikationseinrichtung 37a der Vorrichtung 23 und dem externen System 38 eine drahtgebundene oder bevorzugt drahtlose Datenverbindung 39a (temporär) herstellbar. Vorzugsweise ist zwischen der Kommunikationseinrichtung 37a der Vorrichtung 23 und der Kommunikationseinrichtung 37b des Inhalators 1 eine drahtgebundene oder bevorzugt drahtlose Datenverbindung 39b (temporär) herstellbar. Die Datenverbindungen 39a und 39b sind in Fig. 8 schematisch angedeutet.

Die Kommunikationseinrichtung 37a der Vorrichtung 23 und/oder die Kommunikationseinrichtung 37b des Inhalators 1 sind vorzugsweise dazu ausgebildet, Informationen über den Zustand des Inhalators 1 und/oder dessen Benutzung zu senden und/oder zu empfangen. Diese Informationen können beispielsweise der Zustand bzw. Spannzustand des, insbesondere in die Vorrichtung 23 eingesetzten, Inhalators 1, die Anzahl von Benutzungen oder Auslösungen des Inhalators 1, die Menge von bereits verbrauchtem und/oder noch im Behälter 3 enthaltenen Fluid 2, ein Zählerstand des Inhalators 1, und/oder ein Ladezustand des Inhalators 1 sein. Weiter können diese Informationen Zeitpunkte von und/oder zeitliche Abstände zwischen Spannvorgängen, Benutzungen und/oder Auslösungen des Inhalators 1 sein oder aufweisen.

Das externe System 38 kann beispielsweise ein Computer, insbesondere in Form eines PCs oder Servers oder eines tragbaren Geräts wie eines Smartphones, Tablets oder Laptops, sein. Das externe System 38 kann jedoch auch ein Netzwerk wie etwa das Internet sein, sodass die Informationen über den Zustand des Inhalators 1 über dieses Netzwerk direkt an einen Arzt oder sonstigen medizinischen Betreuer des Benutzers des Inhalators 1 übermittelbar sind.

Auf diese Weise kann die Vorrichtung 23 als Compliance-Schnittstelle (d. h. insbesondere Schnittstelle zur Überprüfung der Befolgung von Vorgaben) ausgebildet sein und/oder zum Patientenmonitoring, zur Patientenüberwachung, Dosisüberwachung und/oder Therapieüberwachung dienen. Die Benutzungen des Inhalators 1 sind auf diese Weise insbesondere registrierbar, speicherbar, verfolgbar und/oder kontrollierbar, sodass insbesondere eine Therapietreue eines Patienten bei einer (verordneten) Benutzung des Inhalators 1 verfolgbar oder überprüfbar ist. Auf diese Weise kann Hilfestellung zur Dosierung erfolgen, beispielsweise durch Ausgabe von Hinweisen oder Erinnerungen, insbesondere durch die Vorrichtung 23.

Ein Datenaustausch zwischen der Kommunikationseinrichtung 37a, 37b des Inhalators 1 und/oder der Vorrichtung 23 und dem externen System 38 kann zum Beispiel über WLAN, WPAN, Bluetooth, Infrarot, NFC, RFID und/oder sonstige geeignete Verfahren bzw. Standards erfolgen. Es ist jedoch auch eine Informationsübertragung bzw. Kommunikation über eine drahtgebundene Datenverbindung 39a möglich.

Vorzugsweise erfolgt die Kommunikation zwischen den Kommunikationseinrichtungen 37a und 37b des Inhalators 1 und der Vorrichtung 23 über WLAN, WPAN, Bluetooth, Infrarot, NFC, RFID und/oder ein sonstiges geeignetes Verfahren und/oder einen sonstigen geeigneten Standard. Es ist jedoch auch möglich, dass die Kommunikation zwischen den Kommunikationseinrichtungen 37a und 37b über eine drahtgebundene Datenverbindung 39b, insbesondere wenn der Inhalator 1 in die Vorrichtung 23 eingesetzt und/oder mit dieser gekoppelt ist, erfolgt.

Vorzugsweise findet eine Datenübertragung bzw. Kommunikation zwischen den Kommunikationseinrichtungen 37a, 37b des Inhalators 1 bzw. der Vorrichtung 23 und/oder zwischen der Kommunikationseinrichtung 37a der Vorrichtung 23 und dem externen System 38 nach einem Einsetzen des Inhalators 1 in die Vorrichtung 23 (automatisch) statt und/oder nur dann, wenn der Inhalator 1 in die Vorrichtung 23 eingesetzt ist bzw. mit dieser gekoppelt ist.

Insbesondere erfolgt eine Kommunikation nur dann, wenn mit der Steuerung 36 und/oder dem Sensor 35 ein Einsetzen oder Koppeln des Inhalators 1 mit der Vorrichtung 23 detektiert wurde. Es ist jedoch (auch) möglich, dass eine Kommunikation zu anderen Zeitpunkten erfolgt, insbesondere auch dann, wenn der Inhalator 1 nicht mit der Vorrichtung 23 gekoppelt oder in diese eingesetzt ist, und/oder dass eine kontinuierliche bzw. ununterbrochene Kommunikation erfolgt.

Es ist auch möglich, dass der Inhalator 1 eine Überwachungseinrichtung, insbesondere eine Überwachungseinrichtung in Form des in der WO 2011/157561 A1 beschriebenen "monitoring device", aufweist. Insbesondere kann die Kommunikationseinrichtung 30a in diesem Fall zur Kommunikation mit dem dort beschriebenen Interface ausgebildet sein bzw. bildet das Interface die Kommunikationseinrichtung 37b des Inhalators 1. Vorzugsweise können über die Datenverbindung 39b die von der Überwachungseinrichtung gespeicherten oder mittels der Überwachungseinrichtung aufgenommenen Daten an die Vorrichtung 23 übermittelt bzw. mit der Vorrichtung 23 ausgetauscht werden. Mit anderen Worten kann die Vorrichtung 23 vorzugsweise dazu ausgebildet sein, die Überwachungseinrichtung auszulesen. Vorzugsweise ist es dabei auch möglich, zu ermitteln, wie viel Zeit zwischen einem Spannen des Inhalators 1 durch die Vorrichtung 23 und einer, insbesondere mit der Überwachungseinrichtung ermittelten, Benutzung des Inhalators 1 vergangen ist.

Der Inhalator 1 und/oder die Vorrichtung 23 kann einen Zähler 40 zum Zählen von Benutzungen des Inhalators 1 aufweisen.

Der Zähler 40 ist vorzugsweise zum Erfassen, Detektieren und/oder Speichern von Benutzungen des Inhalators 1, insbesondere als oder durch Erfassen, Detektieren und/oder Speichern von durch die Vorrichtung 23 erfolgten Spannvorgängen, ausgebildet. Insbesondere sind mittels des Zählers 40 bereits mit einem Behälter 3 abgegebene und/oder noch in einem Behälter 3 enthaltene Dosen der zu zerstäubenden Substanz bzw. des Fluids 2 erfassbar und/oder zählbar. Beispielsweise kann der Zähler 40 wie die in der WO 2005/080001 beschriebene Überwachungseinrichtung ausgebildet sein. Hier sind jedoch auch andere Lösungen möglich.

Der Zähler 40 kann ein mechanischer Zähler sein bzw. ein mechanisches Zählwerk aufweisen, beispielsweise in Form des in der WO 2004/024340 A1 beschriebenen und auch in Fig. 1 angedeuteten Spindel-Zählwerks oder in Form eines oder mehrerer Zählringe mit darauf angebrachten oder aufgetragenen Zahlen zur Anzeige eines Zählerstandes oder Zählerwerts.

Bei einem derartigen mechanischen Zähler 40 kann die Vorrichtung 23 insbesondere dazu ausgebildet sein, den Zähler 40 bzw. Zählerstand oder Zählerwert zu detektieren, auszulesen, abzufragen und/oder zu ermitteln. Dies kann beispielweise über eine optische Detektion eines Anzeigeelements bzw. der Position eines Anzeigeelements, insbesondere über eine Reflexionsmessung, Abstandsmessung, Lasertriangulation und/oder sonstige optische Erfassung, etwa mittels einer Kamera, erfolgen.

Vorzugsweise ist der Zähler 40 jedoch ein elektronischer oder elektromechanischer Zähler.

Der Zähler 40 kann fest in den Inhalator 1 und/oder ein Gehäuseteil 16, 17, 18 des Inhalators 1 integriert und/oder von dem Inhalator 1 abnehmbar ausgebildet sein.

Es ist auch möglich, dass die Vorrichtung 23 den Zähler 40 aufweist.

Bei einer Anordnung das Zählers 40 an oder in der Vorrichtung 23 kann beispielsweise ein Einsetzen des Inhalators 1 in die Vorrichtung 23, ein Entnehmen des Inhalators 1 aus der Vorrichtung 23 und/oder ein veränderter Zustand bzw. Spannzustand des Inhalators 1 zwischen einem Entnehmen und einem nachfolgenden erneuten Einsetzen des Inhalators 1 als Betätigung oder Benutzung des Inhalators 1 ermittelt bzw. gezählt werden.

Es ist auch möglich, dass ein an oder in der Vorrichtung 23 angeordneter Zähler 40 eine Detektionseinrichtung zur Detektion eines Indikators des Inhalators 1 zur Anzeige eines Zählerstands oder Zählerwerts bzw. einer Position eines solchen Indikators aufweist. Ein solcher Indikator kann etwa ein bewegliches Element sein, dessen Position bei jeder Betätigung bzw. Benutzung des Inhalators 1 verändert wird, zum Beispiel ein axial verschiebbares Indikatorelement oder auch ein Zählring, der bei jeder Betätigung bzw. Benutzung des Inhalators 1 weiter gedreht wird. Hier sind jedoch auch andere Lösungen denkbar.

Vorzugsweise weist der Zähler 40 eine Rücksetzeinrichtung zum Zurücksetzen des Zählers auf. Die Rücksetzeinrichtung ist vorzugsweise dazu ausgebildet, einen angezeigten und/oder gespeicherten Zählerwert oder Zählerstand zu verändern, insbesondere zurückzusetzen, zu löschen und/oder auf "0" zu setzen bzw. zu verändern. Das Zurücksetzen des Zählers 40 kann mechanisch, elektronisch und/oder elektromechanisch erfolgen.

Unter einem "Zählerwert" und/oder "Zählerstand" ist insbesondere ein Wert bzw. eine Zahl zu verstehen, die der (vom Zähler 40 erfassten) Anzahl der Benutzungen des Inhalators 1, insbesondere nach einem Zurücksetzen des Zählers 40, entspricht. Auch wenn mit dem Zähler 40 also alle Benutzungen des Inhalators 1 erfassbar sind bzw. erfasst werden, so entspricht der Zählerwert oder Zählerstand vorzugsweise jedoch nur der Anzahl von Benutzungen nach dem letzten Zurücksetzen des Zählers 40. Insbesondere sind die Begriffe "Zählerwert" und "Zählerstand" im Sinne der vorliegenden Erfindung synonym zu verstehen.

Vorzugsweise weist der Zähler 40 ein Betätigungselement auf. Das Betätigungselement ist vorzugsweise dazu ausgebildet und/oder derart angeordnet, dass durch Betätigung des Betätigungselements der Zähler 40 zurücksetzbar ist. Vorzugsweise weist die Rücksetzeinrichtung das Betätigungselement auf.

Das Betätigungselement kann als Taste, Knopf, Schalter und/oder Betätigungsfeld ausgebildet sein, die/der/das manuell von einem nicht dargestellten Benutzer betätigbar, insbesondere drückbar, ist.

Vorzugsweise ist das Betätigungselement mit der Rücksetzeinrichtung gekoppelt und/oder verbunden, bevorzugt sodass durch eine Betätigung des Betätigungselements die Rücksetzeinrichtung gesteuert oder bedient wird bzw. steuerbar oder bedienbar ist. Besonders bevorzugt bewirkt ein Betätigen des Betätigungselements ein Zurücksetzen des Zählers 40 mittels der Rücksetzeinrichtung.

Der Zähler 40 kann dazu ausgebildet sein, eine Anzahl von innerhalb eines dem Zähler 40 vorgegebenen oder vorgebbaren Zeitfensters erfolgten Benutzungen des Inhalators 1 zu ermitteln.

Der Zähler 40 weist vorzugsweise einen Therapieplan bzw. eine Benutzungsvorgabe auf. Im Folgenden sind die Begriffe "Therapieplan" und "Benutzungsvorgabe" synonym zu verstehen, sie bedeuten also im Rahmen der vorliegenden Erfindung das gleiche.

Eine Benutzungsvorgabe ist eine Vorgabe, in der festgehalten ist, wie der Inhalator von dem Benutzer bzw. Patienten zu benutzen ist. Insbesondere gibt eine Benutzungsvorgabe vor, zu welchen Zeitpunkten, in welchen zeitlichen Abständen, wie häufig und/oder in welcher Frequenz, insbesondere innerhalb eines bestimmten Zeitfensters wie etwa einen Tag, ein Patient bzw. Benutzer den Inhalator benutzen soll bzw. mittels des Inhalators ein Medikament inhalieren soll.

Beispielsweise kann eine Benutzungsvorgabe die Vorschrift sein oder aufweisen, dreimal täglich, insbesondere zu festgelegten Zeiten bzw. Zeitpunkten, mit dem Inhalator ein Medikament einzunehmen bzw. zu inhalieren oder viermal täglich jeweils zwei Dosen zu inhalieren. Vorzugsweise wird durch Befolgen des Therapieplans bzw. der Benutzungsvorgabe eine optimale Behandlung bzw. Therapie erreicht oder gewährleistet.

Insbesondere ist unter einer "zu erfolgenden Benutzung" im Sinne der vorliegenden Erfindung eine (noch nicht erfolgte) Benutzung gemäß der Benutzungsvorgabe zu verstehen und/oder eine Benutzung, die gemäß der Benutzungsvorgabe (zukünftig) erfolgen soll bzw. vorgesehen ist.

Vorzugsweise weist der Zähler 40 die Kommunikationseinrichtung 37b auf und/oder ist der Zähler 40 mit der Kommunikationseinrichtung 37b zur Datenübertragung und/oder über eine Datenverbindung mit der Kommunikationseinrichtung 37b und/oder der Kommunikationseinrichtung 37a verbindbar, wie in Fig. 8 angedeutet. Folglich kann der Zähler 40, wenn er im Inhalator 1 vorgesehen ist, über die Kommunikationseinrichtungen 37b, 37a Zählerinformationen an die Vorrichtung 23 übermitteln. Die Vorrichtung 23 wiederum kann die Zählerinformationen speichern, weiterleiten und/oder auswerten. Hierbei kann die Vorrichtung 23 auf Basis der Zählerinformationen Informationen oder ein Signal auszugeben, insbesondere hinsichtlich einer anstehenden und/oder fehlerhaften Benutzung und/oder hinsichtlich eines Status des Inhalators 1, beispielsweise von noch verfügbaren im Behälter 3 vorgehaltenen, Dosen oder dergleichen.

Vorzugsweise ist der Inhalator 1 bzw. dessen Kommunikationseinrichtung 37b zur ausschließlichen Kommunikation mit der Vorrichtung 23 bzw. deren Kommunikationseinrichtung 37a ausgebildet und/oder kommuniziert der Inhalator 1 bzw. dessen Kommunikationseinrichtung 37b nicht direkt mit dem externen System 38, sondern nur mittelbar über die Kommunikationseinrichtung 37a des Inhalators 1 und/oder die Datenverbindungen 39a und 39b. Die Vorrichtung 23 ist diesbezüglich vorzugsweise eine Art Firewall oder Gateway. Die Vorrichtung 23 weist hierbei eine Datenverbindung 39b zum Inhalator 1 auf und nur über die Vorrichtung 23 werden inhalatorbezogene Informationen wie ein Zählerstand extern übermittelt und/oder zum Abruf bereitgestellt. Hier sind jedoch auch andere Lösungen möglich.

Insbesondere sind mit der Kommunikationseinrichtung 37b der Vorrichtung 23 Informationen über (innerhalb des Zeitfensters) erfolgte und/oder zu erfolgende Benutzungen sendbar und/oder empfangbar.

Die Informationen über die (innerhalb des Zeitfensters) erfolgten und/oder zu erfolgenden Benutzungen sind vorzugsweise an die Kommunikationseinrichtung 37a des Inhalators 1 bzw. die Vorrichtung 23 und/oder über die Datenverbindung 39b übertragbar.

Die übertragenen bzw. übertragbaren Informationen können folgendes umfassen bzw. betreffen (nicht-abschließende Aufzählung): Anzahl der innerhalb des Zeitfensters erfolgten Benutzungen und/oder der innerhalb des Zeitfensters zu erfolgenden Benutzungen, Zeitpunkte der Benutzungen, zeitliche Abstände zwischen Benutzungen und/oder Auslösungen des Inhalators 1, Benutzungsvorgabe, Ergebnis eines Abgleichs der Benutzungsvorgabe mit detektierten bzw. gezählten oder erfolgten Benutzungen, Zählerstand, Zählerwert.

Vorzugsweise ist die Vorrichtung 23 bzw. Steuerung 36 dazu ausgebildet, in Abhängigkeit von einem Zählerwert und/oder der von dem Zähler 40 übertragenen Informationen den Antrieb 26 bzw. ein Hinzufügen von Energie bzw. einen Spannvorgang zu steuern, insbesondere in Abhängigkeit des Ergebnisses eines Abgleichs der Benutzungsvorgabe mit erfolgten Benutzungen. Gegebenenfalls ist es auch möglich, dass der Energiespeicher 7 bzw. die Feder nach einem Einsetzen des Inhalators 1 (zunächst) nicht gespannt wird, etwa um eine Überdosierung und/oder eine vorzeitige bzw. von der Benutzungsvorgabe abweichende Benutzung zu verhindern und/oder dass ein Hinzufügen von Energie zu dem Energiespeicher nicht unmittelbar nach dem Einsetzen, sondern erst zu einem späteren Zeitpunkt erfolgt.

Die Vorrichtung 23 weist vorzugsweise eine insbesondere elektronisch betriebene Ausgabeeinrichtung 41 auf, die zur bevorzugt optischen und/oder akustischen Ausgabe von Informationen über den Zustand des Inhalators 1 und/oder dessen Benutzung(en) ausgebildet ist.

Vorzugsweise ist die Ausgabeeinrichtung 41 elektrisch und/oder datentechnisch mit der Steuerung 36 und/oder dem Sensor 35 verbunden und/oder von der Steuerung 36 steuerbar oder gesteuert, insbesondere auf Grundlage von Signalen, die von dem Sensor 35 auf die Steuerung 36 übertragen bzw. an die Steuerung 36 übermittelt werden. Insbesondere erfolgt die Steuerung auf Basis oder unter Berücksichtigung des detektierten Zustands bzw. Spannzustands des Inhalators 1 und/oder des Zählerstands.

Die Ausgabeeinrichtung 41 kann ein Display und/oder einen Lautsprecher und/oder eine Leuchte und/oder einen Vibrationsalarm aufweisen oder durch diese gebildet sein. Die Ausgabeeinrichtung 41 ist in den Figuren nur schematisch angedeutet.

Vorzugsweise sind mit der Ausgabeeinrichtung 41 Informationen, die mit der Kommunikationseinrichtung 37a sendbar und/oder empfangbar sind, ausgebbar bzw. sind mit der Ausgabeeinrichtung 41 entsprechende Signale ausgebbar.

Die Ausgabeeinrichtung 41 bzw. das Display kann beispielsweise ein LCD-Bildschirm, ein LED-Bildschirm oder ein OLED-Bildschirm sein.

Alternativ oder zusätzlich kann die Ausgabeeinrichtung 41 durch ein oder mehrere Leuchtmittel, beispielsweise in Form von LEDs, gebildet sein oder diese aufweisen.

Vorzugsweise ist die Ausgabeeinrichtung 41 dazu ausgebildet, Informationen über den Zustand bzw. Spannzustand des Inhalators 1 und/oder der Vorrichtung 23, einen Ladezustand des Inhalators 1, und/oder Informationen über die bereits abgegebenen und/oder noch vorhandenen oder abgebbaren Dosen des Fluids 2 anzuzeigen bzw. optisch auszugeben. Dies kann beispielsweise durch die Anzeige von Informationstexten, die Anzeige von entsprechenden (abstrakten) Symbolen oder Grafiken, die Anzeige von entsprechenden Zahlen und/oder über eine Farbanzeige erfolgen.

Vorzugsweise kann mittels der von der Ausgabeeinrichtung 41 ausgegebenen bzw. angezeigten Informationen ein nicht dargestellter Benutzer über den Zustand, insbesondere den Spannzustand, des Inhalators 1 informiert werden, sodass eine korrekte Benutzung gewährleistet ist und/oder der Benutzer über etwaige Fehlfunktionen informiert wird.

Mit der Ausgabeeinrichtung 41 können Warnungen und/oder Hinweise ausgegeben werden, beispielsweise wenn eine Einnahme von Medikamenten mittels des Inhalators 1 nicht vorschriftsmäßig bzw. einem Therapieplan entsprechend erfolgt, der Inhalator 1 geladen und/oder der Behälter 3 gewechselt werden muss. Dies ist insbesondere einer Verbesserung der Patienten-Compliance zuträglich.

Die Ausgabeeinrichtung 41 kann auch (zumindest mittelbar) mit dem Sensor 35 verbunden sein bzw. in Kontakt stehen. Insbesondere ist es möglich, dass mit der Ausgabeeinrichtung 41 angezeigt wird, ob der Inhalator 1 vollständig bzw. korrekt in die Vorrichtung 23 eingesetzt ist oder wurde. Es ist möglich, dass ein nicht dargestellter Benutzer mittels eines von der Ausgabeeinrichtung 41 angezeigten Textes dazu aufgefordert wird, den Inhalator 1 erneut, vollständig und/oder korrekt in die Vorrichtung 23 einzusetzen.

Des Weiteren ist es möglich, dass mittels der Ausgabeeinrichtung 41 angezeigt wird, wenn der Inhalator 1 für eine weitere Betätigung oder Benutzung gesperrt ist (beispielsweise durch die weiter oben beschriebene Blockiervorrichtung), wenn der Behälter 3 vorhanden oder nicht vorhanden ist oder dergleichen.

Entsprechende Signale, Warnungen und/oder Hinweise können selbstverständlich sowohl optisch als auch alternativ oder zusätzlich akustisch ausgebbar sein.

Vorzugsweise sind entsprechende Informationen und/oder Warnsignale optisch und alternativ oder zusätzlich über den (optionalen) Lautsprecher ausgebbar. Dies kann beispielsweise über einfache akustische Signale wie ein Piepen oder spezielle Tonfolgen und/oder durch die akustische Ausgabe von vollständigen Sätzen in menschlicher Sprache erfolgen.

Vorzugsweise weist die Vorrichtung 23 eine Energieversorgungseinrichtung 42 zur Versorgung der Vorrichtung 23, insbesondere des Antriebs 26, mit insbesondere elektrischer Energie auf. Die Vorrichtung 23 ist mittels der Energieversorgungseinrichtung 42 elektrisch betreibbar.

Die Energieversorgungseinrichtung 42 kann beispielsweise durch einen Stromanschluss und/oder ein Netzkabel gebildet sein oder diesen/dieses aufweisen. Alternativ oder zusätzlich kann die Energieversorgungseinrichtung 42 eine Batterie, einen Akku und/oder einen sonstigen Energiespeicher aufweisen oder dadurch gebildet sein. Die Energieversorgungseinrichtung 42 ist vorzugsweise an ein Stromnetz anschließbar bzw. hierdurch ladbar. Insbesondere ist der Energiespeicher der Energieversorgungseinrichtung 42 durch Anschließen dieser an ein Stromnetz ladbar.

Die Energieversorgungseinrichtung 42 ist vorzugsweise dazu ausgebildet, den Antrieb 26, den Motor 27, den Sensor 35, die Steuerung 36, die Kommunikationseinrichtung 37a und/oder die Ausgabeeinrichtung 41 mit (elektrischer) Energie bzw. Strom zu versorgen, wie in Fig. 8 schematisch angedeutet. Besonders bevorzugt wird die zum Hinzufügen von Energie zu dem Energiespeicher 7 bzw. Inhalators 1 benötigte Energie mittels der Energieversorgungseinrichtung 42 bereitgestellt.

Mittels des Energiespeichers der Vorrichtung 23 können beispielsweise der Antrieb 26, ein Display und/oder sonstige elektrisch betreibbare Einrichtungen betrieben bzw. mit elektrischer Energie versorgt werden.

Vorzugsweise ist der Energiespeicher der Vorrichtung 23 ein Nickel-Cadmium-Akku, ein Nickel-Metallhydrid-Akku, Lithium-Ionen-Akku, oder ein Lithium-Polymer-Akku. Der Energiespeicher ist vorzugsweise wiederaufladbar. Hier sind jedoch auch andere Lösungen möglich.

Der Energiespeicher kann auch ein Kombinationsspeicher aus einem Akkumulator oder einer Batterie in Kombination mit einem hochkapazitiven Kondensator sein. Diese Kombination hat den Vorteil, dass von einem hochkapazitiven Kondensator in der Regel hohe Entladeströme entnehmbar sind, da dieser einen geringen Innenwiderstand aufweist. Vorzugsweise ist der hochkapazitive Kondensator ein elektronischer Doppelschichtkondensator (EDLC).

Vorzugsweise ist die in dem Energiespeicher der Vorrichtung 23 gespeicherte oder speicherbare Energiemenge ausreichend, um mittels des Antriebs 26 mindestens 60, bevorzugt mindestens 120, insbesondere mindestens 180, besonders bevorzugt mindestens 240 Spannvorgänge des Energiespeichers 7 bzw. der Feder durchzuführen. Ein Spannvorgang ist dabei insbesondere ein vollständiges Spannen der Feder bzw. ein Überführen des Inhalators 1 vom ungespannten (nicht auslösebereiten) Zustand in den gespannten (auslösebereiten) Zustand. Auf diese Weise kann ein Benutzer bzw. Patient die Vorrichtung 23 mehrere Tage oder Wochen, insbesondere mindestens 3, 4 oder 5 Wochen, zum Spannen des Inhalators 1 verwenden, bevor ein Wiederaufladen oder Erneuern des Energiespeichers 34 erforderlich ist.

Der Energiespeicher der Vorrichtung 23 weist vorzugsweise eine Kapazität von mindestens 800 mAh, bevorzugt mindestens 1.000 mAh, insbesondere mindestens 1.500 mAh, besonders bevorzugt mindestens 2.000 mAh, insbesondere bei 3,6 V, 7,2 V oder 10,8 V Nennspannung, auf.

Die Vorrichtung 23 weist vorzugsweise eine Ladeeinrichtung 44 und/oder einen Konnektor zum Laden eines (elektrischen) Energiespeichers 43 des Inhalators 1 auf. So ist die Vorrichtung 23 vorzugsweise als Ladegerät und/oder Ladestation für den Inhalator 1 ausgebildet. Unter einem "elektrischen" Energiespeicher 43 wird hier ein Energiespeicher 43 verstanden, mit dem elektrische Energie speicherbar und/oder abgebbar ist, auch wenn die Speicherung selbst beispielsweise chemisch erfolgt. Insbesondere ist der (elektrische) Energiespeicher 43 nicht mit dem (mechanischen) Energiespeicher 7 zu verwechseln, der beim Darstellungsbeispiel als Feder ausgebildet ist.

Mittels des Energiespeichers 43 des Inhalators 1 können beispielsweise Zähler 40 und/oder sonstige elektrisch betreibbare Einrichtungen des Inhalators 1 betrieben bzw. mit elektrischer Energie versorgt werden.

Vorzugsweise ist der Energiespeicher 43 des Inhalators 1 ein Nickel-Cadmium-Akku, ein Nickel-Metallhydrid-Akku, Lithium-Ionen-Akku, oder ein Lithium-Polymer-Akku. Der Energiespeicher 43 ist vorzugsweise wiederaufladbar. Hier sind jedoch auch andere Lösungen möglich.

Vorzugsweise wird der Energiespeicher 43 des Inhalators 1, insbesondere automatisch, geladen, wenn der Inhalator 1 die Vorrichtung 23 eingesetzt oder mit dieser gekoppelt ist und/oder nachdem ein Einsetzen oder Koppeln des Inhalators 1 - wie oben beschrieben - detektiert wurde.

Der Energiespeicher 43 des Inhalators 1 kann zum Betrieb eines oder mehrerer Sensoren und/oder Zähler 40 des Inhalators 1 ausgebildet sein. Ferner ist alternativ oder zusätzlich bevorzugt, dass der Energiespeicher 43 des Inhalators 1 die Kommunikationseinrichtung 37b des Inhalators 1 mit Energie versorgt. Auf diese Weise kann der Inhalator 1 mittels der Kommunikationseinrichtung 37b Informationen, insbesondere zur Benutzung, zum Zählerstand und/oder zu im Behälter 3 verbleibenden Dosen an die Vorrichtung 23 übermitteln.

Der Energiespeicher 43 des Inhalators 1 kann verhältnismäßig klein sein und beispielsweise nur zum Betrieb des Zählers 40 ausgelegt sein. Die Energie für den Aufbau der Datenverbindung 39a zwischen dem Inhalator 1 und der Vorrichtung 23 kann bei in die Vorrichtung 23 eingesetztem Inhalator 1 direkt von der Vorrichtung 23 an den Inhalator 1 im laufenden Betrieb transferiert werden.

Die Kopplung zwischen dem Inhalator 1 und der Vorrichtung 23 zum Zwecke der Energieversorgung des Inhalators 1 bzw. zum Laden des Energiespeichers 43 mittels der Ladeeinrichtung 44 kann durch galvanischen Kontakt, insbesondere Federkontakte und/oder induktiv erfolgen. In letzterem Fall weist sowohl der Inhalator 1 als auch die Vorrichtung 23 zueinander korrespondierende Induktionsspulen zur Übertragung elektrischer Energie von der Vorrichtung 23 auf den Inhalator 1 auf.

Vorzugsweise weist die Energieversorgungseinrichtung 42 die Ladeeinrichtung 44 auf und/oder ist die Energieversorgungseinrichtung 42 elektrisch mit der Ladeeinrichtung 44 verbunden und/oder verbindbar. Die Ladeeinrichtung 44 kann jedoch auch unabhängig von der Energieversorgungseinrichtung 42 realisiert werden.

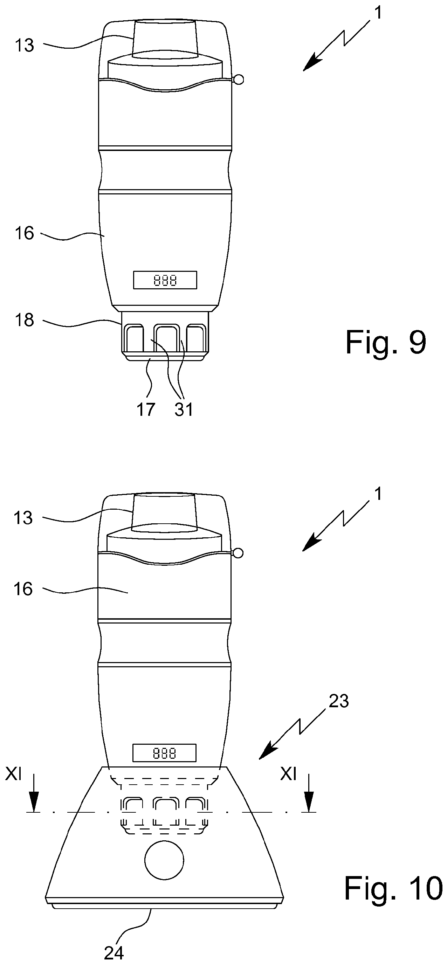

Die Fig. 9 bis 11 zeigen den Inhalator 1 und die Vorrichtung 23 mit dem Inhalator 1 gemäß einer weiteren Ausführungsform. In Fig. 10 sind Teile des Inhalators 1, die von der Vorrichtung 23 verdeckt sind, wenn der Inhalator 1 in die Vorrichtung 23 eingesetzt ist, gestrichelt dargestellt. Nachfolgend werden nur die Unterschiede zu den zuvor erläuterten Ausführungsformen beschrieben, wobei die vorstehenden Erläuterungen vorzugsweise auch für die Ausführungsform aus Fig. 9 bis 11 gelten.

Der Inhalator 1 gemäß der Ausführungsform aus Fig. 9 bis 11 ist vorzugsweise zumindest im Wesentlichen identisch aufgebaut und/oder funktioniert nach demselben Prinzip wie der Inhalator 1 gemäß der ersten Ausführungsform. Vorzugsweise unterscheidet sich der Inhalator 1 gemäß der Ausführungsform aus Fig. 9 bis 11 durch eine andere Gestaltung des Gehäuses bzw. der mechanischen Kopplung mit der Vorrichtung 3 von dem Inhalator 1 gemäß der zuvor beschriebenen ersten Ausführungsform.

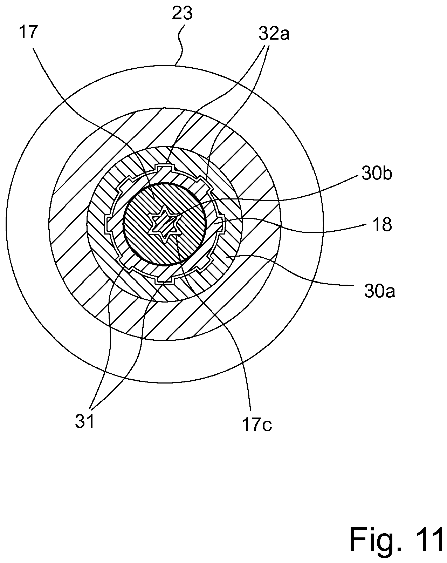

In der Ausführungsform aus Fig. 9 bis 11 weist der Inhalator 1 ein (unteres) Gehäuseteil 18 auf, das durch Einsetzen in oder Koppeln mit der Vorrichtung 23 derart in Eingriff mit der Kopplungseinrichtung 30a kommt, dass der Inhalator 1 in oder an der Vorrichtung 23 gegen Verdrehen gesichert gehalten ist oder wird. Dies wird weiter unten insbesondere anhand von Fig. 11 noch genauer erläutert. Das untere Gehäuseteil 18 kann einstückig mit dem Gehäuseteil 16 ausgebildet sein.

Ferner weist die Vorrichtung 23 vorzugsweise eine Kopplungseinrichtung 30b auf, die bei Einsetzen des Inhalators 1 in oder Koppeln des Inhalators 1 mit der Vorrichtung 23 axial, vorzugsweise formschlüssig, in den Inhalator 1, insbesondere ein inneres Gehäuseteil 17, eingreift und hierdurch eine Kopplung herstellt, über die dem Energiespeicher 7 Energie hinzufügbar bzw. die Feder spannbar ist.

Insbesondere handelt es sich hierbei um eine Steckkopplung, die durch Einsetzen des Inhalators 1 oder Koppeln des Inhalators 1 mit der Vorrichtung 23 automatisch erzeugt wird und über die dem Energiespeicher 7 Energie hinzufügbar bzw. die Feder spannbar ist. Hierbei erzeugt die Vorrichtung 23 vorzugsweise eine Drehbewegung, insbesondere wie zuvor erläutert bzw. mit dem Antrieb 26, die hier jedoch über die Steckkopplung an den Inhalator 1 übertragen wird, der wiederum hierdurch die Feder spannt.

Insbesondere erfolgt eine Kopplung des Antriebs 26 über eine Steckkopplung mit Teilen des Inhalators 1 derart, dass über eine Kulissenführung unter Bewegung des Behälters 3 eine Kompression des Energiespeichers 7 bzw. der Feder erfolgt. Hier sind jedoch auch andere Lösungen denkbar.

Die Steckkopplung kann selbstzentrierend ausgebildet sein, so dass beim Einsetzen des Inhalators 1 in die Vorrichtung 23 ein in der Vorrichtung 23 angeordnetes Gegenstück selbstzentrierend in dem Gehäuseteil 18 aufgenommen wird.

Unter einer Steckkopplung wird eine bevorzugt formschlüssige Verbindung durch axiales ineinander Einführen zweier Teile mit einer gemeinsamen Drehachse verstanden, wodurch ein Drehmoment übertragen werden kann. Nicht zwingend ist hingegen die Übertragbarkeit von Axialkräften, Querkräften, Biegemomenten oder dergleichen. Mit anderen Worten handelt es sich um eine Kupplung zur Übertragung eines Drehmoments von der Vorrichtung 23 auf den Inhalator 1 derart, dass dem Energiespeicher 7 hierdurch Energie hinzufügbar bzw. die Feder spannbar ist. Die Herstellung der Drehmomente übertragenden Verbindung erfolgt vorzugsweise durch Einsetzen bzw. Koppeln des Inhalators 1 mit der Vorrichtung 23.

Eine Steckkopplung kann auch eine Welle-Nabe-Verbindung sein.

Vorzugsweise weist der Inhalator 1 gemäß der Ausführungsform aus Fig. bis 11 keine oder von der ersten Ausführungsform abweichende Kopplungsabschnitte 31 auf. Bei der dargestellten Ausführungsform sind die Kopplungsabschnitte 31 ausschließlich am unteren Gehäuseteil 18 angeordnet. insbesondere weist das Gehäuseteil 18 hier mehr als zwei, vorzugsweise 6 oder 8, Kopplungsabschnitte 31 auf.

Vorzugsweise ist der Inhalator 1 nicht manuell bzw. ausschließlich mittels der Vorrichtung 23 spannbar. Es ist also möglich, dass der Inhalator 1 derart ausgebildet ist, dass ein manuelles Spannen des Inhalators 1 nicht oder nur schwer möglich ist. Dies wird insbesondere dadurch erreicht, dass das untere Gehäuseteil 18 das innere Gehäuseteil 17 überdeckt, dass das untere Gehäuseteil 18 nicht abnehmbar ist, und/oder dass das innere Gehäuseteil 17 nicht oder nur schwer mit den Fingern greifbar und/oder drehbar ist.

Vorzugsweise ist die Vorrichtung 23 dazu ausgebildet, dass beim Einsetzen des Inhalators 1 die erste Kopplungseinrichtung 30a in die Kopplungsabschnitte 31 des Gehäuseteils 18 eingreift und/oder umgekehrt, so dass der Inhalator 1 und/oder das Gehäuseteil 18 drehfest gehalten ist.

Insbesondere ist bei der in den Fig. 9 bis 11 dargestellten Ausführungsform des Inhalators 1 im Gegensatz zu den zuvor erläuterten Ausführungsformen das untere Gehäuseteil 18 nicht drehfest mit dem Innenteil 17 gekoppelt oder koppelbar, sondern gegenüber dem Innenteil 17 verdrehbar bzw. rotierbar.

Vorzugsweise ist das Gehäuseteil 17 des Inhalators 1 dazu ausgebildet, dass mit der Vorrichtung 23 bzw. deren (zweiter) Kopplungseinrichtung 30b, in den Inhalator 1, insbesondere von unten bzw. der Unterseite des Inhalators 1, eingegriffen werden kann, wodurch eine mechanische Kopplung erfolgt.

Die zweite Kopplungseinrichtung 30b ist also vorzugsweise zum Koppeln mit dem inneren Gehäuseteil 17 ausgebildet, insbesondere durch axiales Eingreifen von unten in das Gehäuseteil 17.

Insbesondere ist so eine (direkte) drehfeste Kopplung der zweiten Kopplungseinrichtung 30b der Vorrichtung 23 mit dem drehbaren Innenteil 17 des Inhalators 1 möglich, sodass der Inhalator 1 mittels der Vorrichtung 23 spannbar ist. Dementsprechend ist die Kopplungseinrichtung 30a für den Inhalator 1 nicht an einem oberen Rand 33 der Aufnahme 25 ausgebildet. Stattdessen ist die Kopplungseinrichtung 30a für den Inhalator 1 derart ausgebildet, dass der Inhalator 1 bzw. dessen Gehäuseunterteil 18 formschlüssig mit der Kopplungseinrichtung 30a verbindbar bzw. koppelbar ist. Dementsprechend können auch die Aussparungen 32a der Kopplungseinrichtung 30a entfallen oder anders ausgebildet sein als bei der ersten Ausführungsform (wie in Fig. 11 erkennbar). Insbesondere ergibt sich so im Vergleich zur ersten Ausführungsform eine geringere Bauhöhe der Vorrichtung 23.

Fig. 11 zeigt einen Schnitt durch die Vorrichtung 23 mit darin eingesetztem Inhalator 1 entlang der in Fig. 10 gezeigten Schnittlinie XI-XI.

Die erste Kopplungseinrichtung 30a ist vorzugsweise ringartig ausgebildet und/oder umfasst den in die Vorrichtung 23 eingesetzten Inhalator 1 bzw. dessen Gehäuseteil 18.

Das Innenteil bzw. innere Gehäuseteil 17 ist vorzugsweise zum Gehäuseteil 18 verdrehbar.

Vorzugsweise weist das Gehäuseteil 17 eine Kopplungsaufnahme 17c zur insbesondere drehfesten Kopplung des Gehäuseteils 17 mit der (zweiten) Kopplungseinrichtung 30b der Vorrichtung 23 auf. Die Kopplungsaufnahme 17c ist vorzugsweise an einer Unterseite des Gehäuseteils 17 und/oder zentral bzw. mittig, insbesondere symmetrisch zur Drehachse D, angeordnet. Die Kopplungsaufnahme 17c ist vorzugsweise nach unten offen bzw. bildet vorzugsweise eine nach unten offene Vertiefung oder Öffnung des Gehäuseteils 17.

Die Kopplungsaufnahme 17c bzw. die dadurch gebildete Öffnung ist vorzugsweise so klein, dass nicht per Hand bzw. mit Fingern in die Kopplungsaufnahme 17c eingegriffen werden kann, sodass ein manuelles Drehen des Gehäuseteils 17 durch einen nicht dargestellten Benutzer verhindert oder zumindest deutlich erschwert ist. Vorzugsweise ist der Inhalator 1 gemäß der in den Fig. 9 bis 11 gezeigten Ausführungsform also nicht manuell bzw. nur mittels der Vorrichtung 23 spannbar. Fehlbedienungen können so vermieden werden.

Die Kopplungsaufnahme 17c ist vorzugsweise korrespondierend bzw. komplementär zur Kopplungseinrichtung 30b ausgebildet. Im Darstellungsbeispiel ist die Kopplungsaufnahme 17c sternförmig ausgebildet. Hier sind jedoch auch andere Lösungen möglich, beispielweise eine hexagonale oder quadratische Form oder sonstige zur drehfesten Kopplung geeigneten Ausbildungen.

Dementsprechend ist die Kopplungseinrichtung 30b ist vorzugsweise zur Kopplungsaufnahme 17c korrespondierend, insbesondere ebenfalls sternförmig, hexagonal oder quadratisch, ausgebildet.

Die (zweite) Kopplungseinrichtung 30b ist vorzugsweise mittig bzw. zentral, insbesondere entlang der Drehachse D, in der Vorrichtung 23 bzw. der Aufnahme 25 angeordnet. Besonders bevorzugt ragt die Kopplungseinrichtung 30b axial in Richtung der Drehachse D bzw. nach oben vom Boden 25a der Aufnahme 25 hervor. Insbesondere ist die Kopplungseinrichtung 30b stiftartig oder nippelartig ausgebildet.

Bei dieser Lösung erfolgt also ein Koppeln des Inhalators 1 mit der Vorrichtung ebenfalls durch axiales Einsetzen. Beim Einsetzen wird die erste Kopplungseinrichtung 30a mit dem Gehäuseteil 18 formschlüssig gekoppelt, wobei radial vorstehende, in Umfangsrichtung an dem Gehäuseteil 18 angeordnete Kopplungsabschnitte 31 mit zugeordneten Aussparungen 32a der Kopplungseinrichtung 30a in Eingriff gelangen. Beim Einsetzen wird ebenfalls die zweite Kopplungseinrichtung 30b mit dem inneren Gehäuseteil 17 gekoppelt, wobei beim Einsetzen die stiftartige Kopplungseinrichtung 30b von unten in die Kopplungsaufnahme 17c eingeführt bzw. der Inhalator 1 von oben auf die stiftartige Kopplungseinrichtung 30b aufgesetzt wird. Anschließend kann, wie weiter oben bei der ersten Ausführungsform beschrieben, ein Spannen des Inhalators 1 bzw. Hinzufügen von Energie zu dem Energiespeicher 7 durch Verdrehen der Gehäuseteile 17 und 18 gegeneinander erfolgen.

In einer weiteren, nicht dargestellten Ausführungsform kann der Inhalator 1 - insbesondere zusätzlich zu einem oder mehreren der bisher erläuterten Merkmale und Aspekte - dazu ausgebildet sein, dass eine Auslösung durch ein Einatmen oder eine Inhalation eines Benutzers bzw. Patienten erfolgt. Mit anderen Worten kann der Inhalator 1 also dazu ausgebildet sein, dass eine Atemzugstriggerung erfolgt. Insbesondere kann so das manuelle Auslösen mittels Betätigung der Auslösetaste 8a entfallen.

Der Inhalator 1 weist in dieser weiteren Ausführungsform vorzugsweise ein Detektionsmittel zur Detektion einer Luftströmung, insbesondere eines Einatmens oder einer Inhalation und/oder ein Auslösemittel zum Auslösen der Abgabe einer entsprechenden Dosis auf.

Vorzugsweise weist das Detektionsmittel einen Sensor zur Detektion eines Drucks, eines Druckabfalls, einer Geschwindigkeit, einer Geschwindigkeitszunahme oder eines Wertes auf, der im Zusammenhang mit einem Luftfluss durch den Inhalator 1, insbesondere das Mundstück 13, steht, wenn der Patient einatmet.

Das entsprechende Detektionssignal, das ein Einatmen eines Patienten anzeigt, kann von dem Auslösemittel zum Auslösen einer Abgabe einer entsprechenden Dosis benutzt werden. Mit anderen Worten kann der Inhalator 1 durch oder bei Detektion automatisch ausgelöst werden oder hierzu ausgebildet sein.

Insbesondere kann das Auslösemittel mit dem Druckerzeuger 5 derart gekoppelt sein, dass bei einem Detektionssignal mit dem Druckerzeuger 5 eine Abgabe einer Dosis erfolgt.

Das Auslösemittel kann beispielsweise elektrisch, elektronisch, pneumatisch und/oder mechanisch arbeiten.

Insbesondere kann mit dem Detektionsmittel und/oder dem Auslösemittel ein Atemzugstrigger realisiert sein. Der Atemzugstrigger dient vorzugsweise zur Auslösung basierend auf einer Einatmung durch den Inhalator 1 hindurch bzw. durch einen Luftstrom, der durch den Inhalator 1 bewirkt wird.

Das Detektionsmittel und/oder das Auslösemittel können ein Ventil aufweisen, das durch einen Luftzug, insbesondere beim Einatmen, geöffnet wird. Das Öffnen des Ventils kann dann beispielsweise mechanisch und/oder elektrisch detektiert werden, so dass im Anschluss an die Detektion eine Abgabe einer Dosis erfolgt. Anstelle des Ventils kann auch eine Klappe oder eine Membran vorgesehen sein.

Insbesondere kann vorgesehen sein, dass ein Ventil, eine Klappe und/oder eine Membran derart mechanisch mit dem Druckerzeuger 5 gekoppelt sind, dass bei einem Einatmen eine Abgabe einer Dosis bzw. eine Erzeugung des Aerosols 14 erfolgt bzw. bewirkt wird. Alternativ oder zusätzlich ist jedoch auch eine elektronische Lösung denkbar. Beispielsweise kann ein Drucksensor vorgesehen sein, so dass eine Abgabe einer Dosis bzw. eine Erzeugung des Aerosols 14 erfolgt bzw. bewirkt wird, wenn mit dem Drucksensor ein Einatmen registriert oder gemessen wird.

Die Kommunikationseinrichtung 37b des Inhalators 1 kann dazu ausgebildet sein, Signale des Detektionsmittels und/oder Auslösemittels zu empfangen und insbesondere an die Kommunikationseinrichtung 37a der Vorrichtung 23 weiterzugeben.

Insbesondere erfolgt ein Versetzen des Inhalators 1 in den auslösebereiten Zustand nur in Abhängigkeit von der Benutzung des Inhalators 1, die mit dem Detektionsmittel detektiert wird. Beispielsweise ist es möglich, dass ein (erneutes) Versetzen des Inhalators 1 in den auslösebereiten Zustand nur dann erfolgt, wenn mittels des Detektionsmittels eine vorherige Benutzung des Inhalators 1 detektiert wurde. Eine weitere Möglichkeit besteht darin, dass kein Versetzen des Inhalators 1 in den auslösebereiten Zustand erfolgt, wenn eine Benutzung des Inhalators 1 mit dem Detektionsmittel detektiert wurde, beispielsweise wenn bereits eine vorgegebene oder vorbestimmte Anzahl an vorgesehenen Benutzungen des Inhalators 1 erreicht wurde, etwa eine maximale Tagesdosis.

Vorzugsweise ist durch die Möglichkeit der Auslösung durch Einatmen in Kombination mit dem automatischen Versetzen in den auslösebereiten Zustand mittels der Vorrichtung 23 eine besonders einfache Bedienung bzw. Benutzung des Inhalators 1 ermöglicht. Insbesondere sind so weder ein manuelles Vorbereiten des Inhalators 1 zur Auslösung noch ein manuelles Auslösen nötig, sondern der Inhalator kann aus der Vorrichtung 23 entnommen werden, die Auslösung erfolgt automatisch beim Einatmen und anschließend wird der Inhalator wieder in die Vorrichtung 23 eingesetzt. Eine Benutzung des Inhalators ist so denkbar einfach gestaltet und kann kaum noch fehlerhaft erfolgen.

**Bezugszeichenliste:**

| | | | |
|---|---|---|---|
| 1 | Inhalator | 26 | Antrieb |
| 2 | Fluid | 27 | Motor |
| 3 | Behälter | 28 | Getriebe |
| 4 | Fluidraum | 29a | (erstes) Zahnrad |
| 5 | Druckerzeuger | 29b | (zweites) Zahnrad |
| 6 | Halterung | 30a | Kopplungseinrichtung |
| 7 | Energiespeicher | 30b | Kopplungseinrichtung |
| 8 | Sperrelement | 31 | Kopplungsabschnitt |
| 8a | Auslösetaste | 32a | Aussparung |
| 9 | Förderrohr | 32b | Aussparung |
| 10 | Rückschlagventil | | |
| 11 | Druckkammer | 33 | Rand |
| 12 | Austragsdüse | 34 | Zähne |
| 13 | Mundstück | 35 | Sensor |
| 14 | Aerosol | 36 | Steuerung |
| 15 | Zuluftöffnung | 37a | Kommunikationseinrichtung |
| 16 | Gehäuseteil | | (Vorrichtung) |
| 16a | Abflachungen | 37b | Kommunikationseinrichtung |
| 17 | Innenteil | | (Inhalator) |
| 17a | oberer Teil (Innenteil) | 38 | externes System |
| 17b | unterer Teil (Innenteil) | 39a | Datenverbindung |
| 17c | Kopplungsaufnahme | 39b | Datenverbindung |
| 18 | (unteres) Gehäuseteil | 40 | Zähler |
| 19 | Halteelement | 41 | Ausgabeeinrichtung |
| 20 | Feder | 42 | Energieversorgungseinrichtung |
| 21 | Behälterboden | | |
| 22 | Anstechelement | 43 | Energiespeicher |
| 23 | Vorrichtung | 44 | Ladeeinrichtung |
| 24 | Standfläche | | |
| 25 | Aufnahme | D | Drehachse |
| 25a | Boden | | |

## Patentansprüche

1. Portable Vorrichtung (23) zum Versetzen eines Inhalators (1) in einen auslösebereiten Zustand durch Hinzufügen von Energie zu einem mechanischen Energiespeicher (7) des Inhalators (1), wobei der Inhalator (1) zum Versetzen in den auslösebereiten Zustand in die Vorrichtung (23) einsetzbar und/oder mit der Vorrichtung (23) mechanisch koppelbar ist, wobei die Vorrichtung (23) manuell und ohne Hilfsmittel von einem Benutzer bzw. Patienten transportierbar ist,
wobei der Inhalator (1) zur Benutzung nicht aus der Vorrichtung (23) entnommen werden muss und der Inhalator (1) gemeinsam mit der Vorrichtung (23) genommen, vom Benutzer bzw. Patienten manuell bewegt bzw. hochgehoben, und zur Inhalation zum Mund des Patienten geführt werden kann,
wobei die Vorrichtung (23) einen motorischen Antrieb (26) aufweist, wobei der Antrieb (26) zum relativen Bewegen zweier Gehäuseteile (16, 17, 18) des in die Vorrichtung (23) eingesetzten und/oder mit der Vorrichtung (23) gekoppelten Inhalators (1) zueinander ausgebildet ist, wobei durch die Bewegung der Gehäuseteile (16, 17, 18) zueinander dem Energiespeicher (7) Energie mechanisch hinzufügbar ist;
**dadurch gekennzeichnet, dass** die Vorrichtung (23) und/oder der Antrieb (26) dazu ausgebildet sind, die Gehäuseteile (16, 17, 18) gegeneinander zu verdrehen.

2. Vorrichtung nach Anspruch 1, **dadurch gekennzeichnet, dass** die Vorrichtung (23) und/oder der Antrieb (26) dazu ausgebildet sind, die Gehäuseteile (16, 17, 18) um mehr als 90° und/oder um höchstens 360°, insbesondere um 180°, gegeneinander zu verdrehen.

3. Vorrichtung nach einem der voranstehenden Ansprüche, **dadurch gekennzeichnet, dass** die Vorrichtung (23) zwei Kopplungseinrichtungen (30a, 30b) aufweist,
wobei die Kopplungseinrichtungen (30a, 30b) jeweils mit einem von zwei Gehäuseteilen (16, 17, 18) des Inhalators (1) vorzugsweise mechanisch koppelbar sind, so dass mit den Kopplungseinrichtungen (30a, 30b) die zwei Gehäuseteile (16, 17, 18) relativ zueinander bewegbar sind.

4. Vorrichtung nach einem der voranstehenden Ansprüche, **dadurch gekennzeichnet, dass** die Vorrichtung (23) und/oder der Antrieb (26) dazu ausgebildet sind, ein Hinzufügen von Energie zu dem Energiespeicher (7) beim Erreichen eines vorgegebenen Maximaldrehmoments zu stoppen, vorzugsweise wobei das Maximaldrehmoment 0,5 Nm, 0,6 Nm oder 0,8 Nm beträgt.

5. Vorrichtung einem der voranstehenden Ansprüche, **dadurch gekennzeichnet, dass** der Antrieb (26) einen elektrischen Motor (27) und/oder ein Getriebe (28) aufweist.

6. Vorrichtung nach einem der voranstehenden Ansprüche, **dadurch gekennzeichnet, dass** die Vorrichtung (23) einen Sensor (35) aufweist, der dazu ausgebildet ist, das Einsetzen, das Koppeln und/oder den Zustand bzw. Spannzustand des Inhalators (1) zu detektieren.

7. Vorrichtung nach Anspruch 6, **dadurch gekennzeichnet, dass** die Vorrichtung (23) eine Steuerung (36) aufweist, die dazu ausgebildet ist, mit dem Sensor (35) das Einsetzen und/oder Koppeln des Inhalators (1) und dessen Zustand bzw. Spannzustand zu detektieren und in Abhängigkeit hiervon den Antrieb (26) zu steuern, vorzugsweise sodass der Inhalator (1) automatisch durch Hinzufügen von Energie zu dem Energiespeichers (7) in den auslösebereiten Zustand versetzt wird.

8. Vorrichtung nach einem der voranstehenden Ansprüche, **dadurch gekennzeichnet, dass** die Vorrichtung (23) eine Kommunikationseinrichtung (37a) zur vorzugsweise drahtlosen Kommunikation mit dem Inhalator (1) und/oder einem externen System (38) aufweist, vorzugsweise wobei die Kommunikationseinrichtung (37a) dazu ausgebildet ist, Informationen über den Zustand des Inhalators (1) und/oder dessen Benutzung zu senden und/oder zu empfangen.

9. Vorrichtung nach einem der voranstehenden Ansprüche, **dadurch gekennzeichnet, dass** die Vorrichtung (23) eine vorzugsweise elektronisch betriebene Ausgabeeinrichtung (41) zur bevorzugt optischen und/oder akustischen Ausgabe von Informationen über den Zustand des Inhalators (1) und/oder dessen Benutzung aufweist.

10. Vorrichtung nach einem der voranstehenden Ansprüche, **dadurch gekennzeichnet, dass** die Vorrichtung (23) eine Energieversorgungseinrichtung (42) zur Versorgung der Vorrichtung (23) mit Energie und/oder eine Ladeeinrichtung (44) zum Laden eines elektrischen Energiespeichers (43) des Inhalators (1) aufweist.

11. Vorrichtung nach einem der voranstehenden Ansprüche, **dadurch gekennzeichnet, dass** die Vorrichtung (23) eine Aufnahme (25) zum Einsetzen und/oder Halten des Inhalators (1) aufweist, vorzugsweise wobei die Vorrichtung (23) dazu ausgebildet ist, dass durch Einsetzen des Inhalators (1) in die Aufnahme (25) der Antrieb (26) mit einem der Gehäuseteile (16, 17, 18) verbunden wird und das andere Gehäuseteil (16, 17, 18) vorzugsweise formschlüssig mit der Aufnahme (25) verbunden wird, so dass das mit dem Antrieb (26) verbundene Gehäuseteil (16, 17, 18) relativ zu dem anderen Gehäuseteil (16, 17, 18) bewegbar, vorzugsweise drehbar, ist und das andere Gehäuseteil (16, 17, 18) - zumindest während des Spannens - unbeweglich, vorzugsweise drehfest, in der Aufnahme (25) gehalten ist.

12. Vorrichtung nach einem der voranstehenden Ansprüche, **dadurch gekennzeichnet, dass** die Vorrichtung (23) als Dockingstation und/oder Ladestation für den Inhalator (1) ausgebildet ist.

13. Vorrichtung nach einem der voranstehenden Ansprüche, **dadurch gekennzeichnet, dass** die Vorrichtung (23) eine Kommunikationseinrichtung (37a) zur bevorzugt drahtlosen Kommunikation mit einem externen System (38) und/oder mit dem Inhalator (1), insbesondere einer Kommunikationseinrichtung (37b) des Inhalators (1), aufweist.

14. Vorrichtung nach einem der voranstehenden Ansprüche, **dadurch gekennzeichnet, dass** die Vorrichtung (23) als Compliance-Schnittstelle ausgebildet ist und/oder zum Patientenmonitoring, zur Patientenüberwachung, Dosisüberwachung und/oder Therapieüberwachung dient.

15. System mit einer Vorrichtung (23) nach einem der voranstehenden Ansprüche und einem Inhalator (1), wobei durch die Vorrichtung (23) einem Energiespeicher (7) des Inhalators (1) durch eine relative Bewegung zweier Gehäuseteile (16, 17, 18) des Inhalators (1) zueinander Energie hinzufügbar ist.

16. System nach Anspruch 15, **dadurch gekennzeichnet, dass** der Inhalator (1) einen Zähler (40) zum Zählen von Benutzungen des Inhalators (1) aufweist.

17. System nach Anspruch 15 oder 16, **dadurch gekennzeichnet, dass** die Vorrichtung (23) und der Inhalator (1) jeweils eine Kommunikationseinrichtung (37a, 37b) zur vorzugsweise drahtlosen Kommunikation aufweisen, so dass eine Datenverbindung (39b) zwischen den Kommunikationseinrichtungen (37a, 37b) der Vorrichtung (23) und des Inhalators (1) herstellbar ist, vorzugsweise wobei der Zähler (40) über eine Datenverbindung mit einer der Kommunikationseinrichtungen (37a, 37b) verbindbar ist.

18. System nach einem der Ansprüche 15 bis 17, **dadurch gekennzeichnet, dass** der Inhalator (1) einen mechanischen Druckerzeuger (5) zur Förderung und Zerstäubung eines Fluids (2) aufweist, wobei das Fluid (2) bei Hinzufügen von Energie zu dem Energiespeicher (7) in eine Druckkammer (11) des Druckerzeugers (5) gefördert wird, und/oder wobei durch Entspannung des Energiespeichers (7) das Fluid (2) unter Druck gesetzt und über eine Austragsdüse (12) als einatembares Aerosol (14) ausgegeben wird.

## Claims

1. Portable device (23) for putting an inhaler (1) into a triggering-ready state by adding energy to a mechanical energy store (7) of the inhaler (1), wherein in order to put the inhaler (1) into the triggering-ready state the inhaler can be inserted into the device (23) and/or mechanically coupled to the device (23), wherein the device (23) can be manually transported by a user or patient by hand without aids, wherein the inhaler (1) does not have to be removed from the device (23) in order to use it and wherein the inhaler (1) can be taken in combination with the device (23), can manually be moved or lifted by a user or patient and brought to the patient's mouth for the inhalation,
wherein the device (23) comprises a motor drive (26), wherein the drive (26) is designed for the relative movement of two housing parts (16, 17, 18) of the inhaler (1) inserted into the device (23) and/or coupled to the device (23), with respect to one another, wherein by the movement of the housing parts (16, 17, 18) relative to one another energy can be added mechanically to the energy store (7), **characterised in that** the device (23) and/or the drive (26) are designed to rotate the housing parts (16, 17, 18) relative to one another.

2. Device according to claim 1, **characterised in that** the device (23) and/or the drive (26) are designed to rotate the housing parts (16, 17, 18) by more than 90° and/or by at most 360°, in particular by 180°, relative to one another.

3. Device according to one of the preceding claims, **characterised in that** the device (23) comprises two coupling devices (30a, 30b), wherein the coupling devices (30a, 30b) can each be preferably mechanically coupled to one of two housing parts (16, 17, 18) of the inhaler (1) so that the two housing parts (16, 17, 18) can be moved relative to one another with the coupling devices (30a, 30b).

4. Device according to one of the preceding claims, **characterised in that** the device (23) and/or the drive (26) are designed to stop an addition of energy to the energy store (7) when a predetermined maximum torque is reached, preferably wherein the maximum torque is 0.5 Nm, 0.6 Nm or 0.8 Nm.

5. Device according to one of the preceding claims, **characterised in that** the drive (26) comprises an electric motor (27) and/or a gear mechanism (28).

6. Device according to one of the preceding claims, **characterised in that** the device (23) comprises a sensor (35), which is designed to detect the insertion, coupling and/or the state or tension state of the inhaler (1).

7. Device according to claim 6, **characterised in that** the device (23) comprises a controller (36), which is designed to detect with the sensor (35) the insertion and/or coupling of the inhaler (1) and its state or tension state, and depending on this to control the drive (26), preferably so that the inhaler (1) is automatically put into the triggering-ready state by addition of energy to the energy store (7).

8. Device according to one of the preceding claims, **characterised in that** the device (23) comprises a communication device (37a) for the preferably wireless communication with the inhaler (1) and/or an external system (38), preferably wherein the communication device (37a) is designed to send and/or receive information on the state of the inhaler (1) and/or its use.

9. Device according to one of the preceding claims, **characterised in that** the device (23) comprises a preferably electronically operated output device (41) for the preferably optical and/or acoustic output of information on the condition of the inhaler (1) and/or its use.

10. Device according to one of the preceding claims, **characterised in that** the device (23) comprises an energy supply device (42) for supplying the device (23) with energy and/or a charging device (44) for charging an electrical energy store (43) of the Inhaler (1).

11. Device according to one of the preceding claims, **characterised in that** the device (23) comprises a receptacle (25) for the insertion and/or retention of the inhaler (1), preferably wherein the device (23) is designed so that by inserting the inhaler (1) into the receptacle (25), the drive (26) is connected to one of the housing parts (16, 17, 18) and the other housing part (16, 17, 18) is preferably positively connected to the receptacle (25), so that the housing part (16, 17, 18) connected to the drive (26) can be moved, preferably rotated, relative to the other housing part (16, 17, 18), and the other housing part (16, 17, 18) is held - at least during tensioning - immovably, preferably in a rotation-proof manner, in the receptacle (25).

12. Device according to one of the preceding claims, **characterised in that** the device (23) is designed as a docking station and/or charging station for the inhaler (1).

13. Device according to one of the preceding claims, **characterised in that** the device (23) comprises a communication device (37a) for the preferably wireless communication with an external system (38) and/or with the inhaler (1), in particular with a communication device (37b) of the inhaler (1).

14. Device according to one of the preceding claims, **characterised in that** the device (23) is designed as a compliance interface and/or serves for patient monitoring, patient supervision, dose monitoring and/or treatment supervision.

15. System with a device (23) according to one of the preceding claims and an inhaler (1), wherein by means of the device (23) energy can be added to an energy store (7) of the inhaler (1) by a relative movement of two housing parts (16, 17, 18) of the inhaler (1) with respect to one another.

16. System according to claim 15, **characterised in that** the inhaler (1) comprises a counter (40) for counting uses of the inhaler (1).

17. System according to claim 15 or 16, **characterised in that** the device (23) and the inhaler (1) each comprise a communication device (37a, 37b) for the preferably wireless communication, so that a data connection (39b) can be established between the communication devices (37a, 37b) of the device (23) and the inhaler (1), preferably wherein the counter (40) can be connected via a data connection to one of the communication devices (37a, 37b).

18. System according to any one of claims 15 to 17, **characterised in that** the inhaler (1) comprises a mechanical pressure generator (5) for conveying and atomising a fluid (2), wherein the fluid (2) on addition of energy to the energy store (7) is conveyed to a pressure chamber (11) of the pressure generator (5), and/or wherein by detensioning the energy store (7) the fluid (2) is pressurised and is discharged via a discharge nozzle (12) as an inhalable aerosol (14).

## Revendications

1. Dispositif portable (23) pour commuter un inhalateur (1) dans un état prêt pour le déclenchement par addition d'énergie à un accumulateur d'énergie (7) mécanique de l'inhalateur (1), dans lequel l'inhalateur (1) à commuter dans l'état prêt pour le déclenchement peut être inséré dans le dispositif (23) et/ou peut être couplé mécaniquement au dispositif (23), dans lequel
le dispositif (23) peut être transporté manuellement et sans accessoires par un utilisateur ou un patient, dans lequel l'inhalateur (1) ne doit pas être retiré du dispositif (23) pour utilisation et l'inhalateur (1) peut être pris conjointement avec le dispositif (23), peut être déplacé ou relevé manuellement par l'utilisateur ou le patient et peut être porté à la bouche du patient pour l'inhalation,
dans lequel le dispositif (23) présente un entraînement (26) motorisé, dans lequel l'entraînement (26) est réalisé pour déplacer de manière relative l'une par rapport à l'autre deux parties de boîtier (16, 17, 18) de l'inhalateur (1) inséré dans le dispositif (23) et/ou couplé au dispositif (23), dans lequel l'addition d'énergie à l'accumulateur d'énergie (7) peut être effectuée mécaniquement par le déplacement l'une par rapport à l'autre des parties de boîtier (16, 17, 18) ;
**caractérisé en ce que** le dispositif (23) et/ou l'entraînement (26) sont réalisés pour faire tourner l'une à l'encontre de l'autre les parties de boîtier (16, 17, 18).

2. Dispositif selon la revendication 1,
**caractérisé en ce que** le dispositif (23) et/ou l'entraînement (26) sont réalisés pour faire tourner l'une à l'encontre de l'autre les parties de boîtier (16, 17, 18) de plus de 90° et/ou de 360° au maximum, en particulier de 180°.

3. Dispositif selon l'une quelconque des revendications précédentes, **caractérisé en ce que** le dispositif (23) présente deux systèmes de couplage (30a, 30b),
dans lequel les systèmes de couplage (30a, 30b) peuvent être couplés mécaniquement de préférence respectivement à une des deux parties de boîtier (16, 17, 18) de l'inhalateur (1) de sorte que les deux parties de boîtier (16, 17, 18) peuvent être déplacées de manière relative l'une par rapport à l'autre avec les systèmes de couplage (30a, 30b).

4. Dispositif selon l'une quelconque des revendications précédentes, **caractérisé en ce que** le dispositif (23) et/ou l'entraînement (26) sont réalisés pour arrêter une addition d'énergie à l'accumulateur d'énergie (7) lorsqu'un couple de rotation maximal spécifié est atteint, de préférence dans lequel le couple de rotation maximal est de 0,5 Nm, de 0,6 Nm ou de 0,8 Nm.

5. Dispositif selon l'une quelconque des revendications précédentes, **caractérisé en ce que** l'entraînement (26) présente un moteur électrique (27) et/ou une transmission (28).

6. Dispositif selon l'une quelconque des revendications précédentes, **caractérisé en ce que** le dispositif (23) présente un capteur (35), qui est réalisé pour détecter l'insertion, le couplage et/ou l'état ou l'état de serrage de l'inhalateur (1).

7. Dispositif selon la revendication 6, **caractérisé en ce que** le dispositif (23) présente une commande (36) qui est réalisée pour détecter avec le capteur (35) l'insertion et/ou le couplage de l'inhalateur (1) et son état ou son état de serrage et pour commander en fonction de celui-ci l'entraînement (26), de préférence de sorte que l'inhalateur (1) est commuté automatiquement par l'addition d'énergie à l'accumulateur d'énergie (7) dans l'état prêt pour le déclenchement.

8. Dispositif selon l'une quelconque des revendications précédentes, **caractérisé en ce que** le dispositif (23) présente un système de communication (37a) pour communiquer de préférence sans fil avec l'inhalateur (1) et/ou un système externe (38), de préférence dans lequel le système de communication (37a) est réalisé pour envoyer et/ou recevoir des informations sur l'état de l'inhalateur (1) et/ou son utilisation.

9. Dispositif selon l'une quelconque des revendications précédentes, **caractérisé en ce que** le dispositif (23) présente un système d'envoi (41) fonctionnant de préférence de manière électronique pour l'envoi de préférence optique et/ou acoustique d'informations sur l'état de l'inhalateur (1) et/ou son utilisation.

10. Dispositif selon l'une quelconque des revendications précédentes, **caractérisé en ce que** le dispositif (23) présente un système d'alimentation en énergie (42) pour alimenter le dispositif (23) en énergie et/ou un système de charge (44) pour charger un accumulateur d'énergie (43) électrique de l'inhalateur (1) .

11. Dispositif selon l'une quelconque des revendications précédentes, **caractérisé en ce que** le dispositif (23) présente un logement (25) pour insérer et/ou maintenir l'inhalateur (1), de préférence dans lequel le dispositif (23) est réalisé pour que l'entraînement (26) soit relié à une des parties de boîtier (16, 17, 18) par l'insertion de l'inhalateur (1) dans le logement (25) et l'autre partie de boîtier (16, 17, 18) soit reliée de préférence par complémentarité de forme au logement (25) de sorte que la partie de boîtier (16, 17, 18) reliée à l'entraînement (26) puisse être déplacée, de préférence puisse être tournée, par rapport à l'autre partie de boîtier (16, 17, 18), et l'autre partie de boîtier (16, 17, 18) est maintenue de manière immobile, de préférence de manière solidaire en rotation, dans le logement (25) - au moins pendant le serrage -.

12. Dispositif selon l'une quelconque des revendications précédentes, **caractérisé en ce que** le dispositif (23) est réalisé en tant que station d'amarrage et/ou station de charge pour l'inhalateur (1) .

13. Dispositif selon l'une quelconque des revendications précédentes, **caractérisé en ce que** le dispositif (23) présente un système de communication (37a) pour la communication de manière préférée sans fil avec un système externe (38) et/ou avec l'inhalateur (1), en particulier un système de communication (37b) de l'inhalateur (1).

14. Dispositif selon l'une quelconque des revendications précédentes, **caractérisé en ce que** le dispositif (23) est réalisé en tant qu'interface de conformité et/ou pour le suivi du patient, pour la surveillance du patient, pour la surveillance du dosage et/ou pour la surveillance de la thérapie.

15. Système avec un dispositif (23) selon l'une quelconque des revendications précédentes et un inhalateur (1), dans lequel l'addition d'énergie à un accumulateur d'énergie (7) de l'inhalateur (1) peut être effectuée par le dispositif (23) par un déplacement relatif de deux parties de boîtier (16, 17, 18) de l'inhalateur (1) l'une par rapport à l'autre.

16. Système selon la revendication 15, **caractérisé en ce que** l'inhalateur (1) présente un compteur (40) pour compter des utilisations de l'inhalateur (1).

17. Système selon la revendication 15 ou 16, **caractérisé en ce que** le dispositif (23) et l'inhalateur (1) présentent respectivement un système de communication (37a, 37b) pour la communication de préférence sans fil si bien qu'une liaison de données (39b) peut être établie entre les systèmes de communication (37a, 37b) du dispositif (23) et l'inhalateur (1), de préférence dans lequel le compteur (40) peut être relié par l'intermédiaire d'une liaison de données à un des systèmes de communication (37a, 37b) .

18. Système selon l'une quelconque des revendications 15 à 17, **caractérisé en ce que** l'inhalateur (1) présente un générateur de pression mécanique (5) pour refouler et atomiser un fluide (2), dans lequel le fluide (2) est refoulé dans une chambre de pression (11) du générateur de pression (5) lors de l'addition d'énergie à l'accumulateur d'énergie (7), et/ou dans lequel le fluide (2) est mis sous pression par la détente de l'accumulateur d'énergie (7) et est distribué en tant qu'aérosol inhalable (14) par l'intermédiaire d'une buse de décharge (12).
